# EUROPEAN PATENT SPECIFICATION

(11) **EP 3 072 877 B1**
(45) Date of publication and mention of the grant of the patent: **15.08.2018**
(21) Application number: 14875301.5
(22) Date of filing: 26.12.2014
(51) Int. Cl.: C07C 69/14, C07C 69/24, C11B 9/00, C11D 3/20, C11D 3/50, C11D 11/00, A61K 8/37, A61Q 19/00

(54) **ESTER**
ESTER
ESTER

(30) Priority: 26.12.2013 JP 2013268775
(43) Date of publication of application: 28.09.2016
(73) Proprietor: Kao Corporation, Chuo-ku Tokyo 103-8210 (JP)
(72) Inventor: MISHIRO, Asami, Wakayama-shi Wakayama 640-8580 (JP); UEDA, Junko, Wakayama-shi Wakayama 640-8580 (JP); ASADA, Takahiro, Wakayama-shi Wakayama 640-8580 (JP); SATO, Makiko, Tokyo 131-8501 (JP)
(74) Representative: Hoffmann Eitle
(86) International application number: PCT/JP2014/084531
(87) International publication number: WO 2015/099130

(56) References cited:
- EP-A1- 0 003 361
- WO-A1-2013/180224
- JP-A- 2008 094 731
- US-A- 2 867 668
- US-A- 3 452 105
- US-A- 3 549 714
- BUELENS, A.: "Sur L'Ethylisoamyl Carbinol et le Methylisohexyl Carbinol", RECUEIL DES TRAVAUX CHIMIQUES DES PAYS-BAS ET DE LA BELGIQUE, [Online] vol. 28, 1 January 1909 (1909-01-01), - 1 January 1909 (1909-01-01), pages 113-119, XP002768703,
- GOSEI KORYO KAGAKU TO SHOHIN CHISHIKI 06 March 1996, pages 591 - 618, XP008184244

## Description

### Technical Field

The present invention relates a new ester and a fragrance composition containing the ester.

### Background Art

Fragrance is an important element that produces, e.g., preference, a sense of luxury, a sense of ease, and expectations for the effect of products and the like. Furthermore, a distinctive fragrance provides a product differentiation effect and the capacity for attracting customers. Particularly, floral fragrance notes are preferred for toiletry products.

Esters are formed by the reaction of carboxylic acids with various alcohols, and have a large number of variations. Moreover, the esters are known as important fragrance materials. Fragrant esters are described, for example, by A. Buelens in Recueil Des Travaux Chimiques Des Pays-Bas et de la Belgique, 1909, pp. 113-119.

Among the esters, there are many types of esters that are derived from an acetic acid and an acyclic aliphatic alcohol with a branched chain. Examples of such esters include isononyl acetate and octenyl acetate. The isononyl acetate has a woody and fruity odor. The octenyl acetate smells of mint, fruits, and fresh herbs reminiscent of lavender and lavandin. See Non-Patent Document 1.

Linalyl acetate is known to have a fresh, sweet, fruity, and floral odor (see Non-Patent Document 2). Tetrahydrolinalyl acetate is known to have a fresh, floral, fruity, and dry odor (see Non-Patent Document 3).

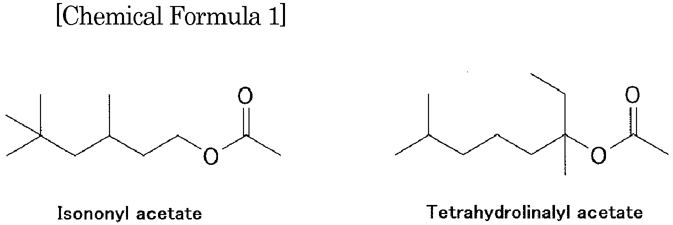

Patent Document 1 discloses that, e.g., 3-acetoxy-4-ethyl-7-methyloctane has a very fresh, herby, fruity, and slightly floral odor.

Patent Document 2 discloses that an isomeric mixture of tetrahydro allo-ocimenyl acetate has the following fractions: a fraction with an odor like that of anise and basil; a fraction with an odor like that of soft linalyl acetate; a fraction with an odor like that of terpinyl acetate; and a fraction with an odor like that of disagreeable butyric acid.

The fragrance materials with similar structures may have roughly similar fragrance notes. However, there are many exceptions. In particular, if the structure is changed by combining a plurality of substituents, it is difficult to predict how the fragrance notes will change, and it is also difficult to predict how they will harmonize with other fragrance materials.

### Prior Art Documents

### Patent Documents

Patent Document 1: US 3,452,105
Patent Document 2: US 2,867,668

### Non-Patent Documents

Non-Patent Document 1: "Basic Knowledge of Perfumes and Perfume Blending" written and edited by Motoki NAKAJIMA, p. 214 and p. 482 (1995)
Non-Patent Document 2: "Synthetic Perfumes, Chemistry and Commodity Knowledge" written by Motoichi INDO, enlarged and revised edition, p. 491 (2005)
Non-Patent Document 3: "Givaudan Roure Index 1998" (Givaudan product catalog), p. 155 (1998)

### Disclosure of Invention

### Problem to be Solved by the Invention

To increase the flexibility in perfume blending, a wide variety of fragrance materials is desired. In particular, there has been a demand for a fragrance composition that has a useful, fresh, and fruity fragrance note and is suitable for perfuming toiletry products.

It is an object of the present invention to provide a new ester that has a fresh and fruity fragrance note useful as a fragrance material, and a fragrance composition containing the ester.

### Means for Solving Problem

The present inventors found out that an ester with a specific structure has a fresh and fruity fragrance note, and thus completed the present invention.

The present invention is directed to an ester represented by the formula (I) and a fragrance composition containing the ester represented by the formula (I).

### Effects of the Invention

The ester of the present invention has a fresh and fruity fragrance note useful as a fragrance material.

### Description of the Invention

### [Ester]

An ester of the present invention is represented by the following formula (I) wherein R¹ represents methyl, ethyl, propyl, or isopropyl, and wherein both R² and R³ represent methyl. The scope of protection sought for this ester, for a fragrance composition comprising the ester and various applications thereof, and for the use of the ester or its method of production, is defined by the appended claims.

To provide suitable context for the present invention, and to help define additional esters that might be used together with the ester of the present invention, the following disclosure refers to esters represented by formula (I) where R¹ represents methyl, ethyl, propyl, or isopropyl, R² represents methyl or ethyl, and R³ represents methyl or hydrogen, with the proviso that when R² is methyl, R³ is methyl and with the proviso that when R² is ethyl, R³ is hydrogen (which may be referred to as an "ester of the formula (I)" or an "ester (I)" in the present specification).

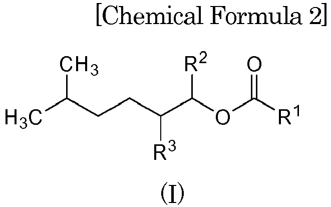

The compound of the formula (I) in which R² is methyl and R³ is methyl (i.e. an ester of the present invention) is also represented by the following formula (I-a). The compound of the formula (I) in which R² is ethyl and R³ is hydrogen (and provided for reference purposes) is also represented by the following formula (I-b). The ester represented by the formula (I-a) may be referred to as an "ester (I-a)" or a "compound (I-a)" in the present specification. The ester represented by the formula (I-b) may be referred to as an "ester (I-b)" or a "compound (I-b) in the present specification. The "compound (I)" includes the "compound (I-a)" and the "compound (I-b)" unless otherwise noted in the present specification.

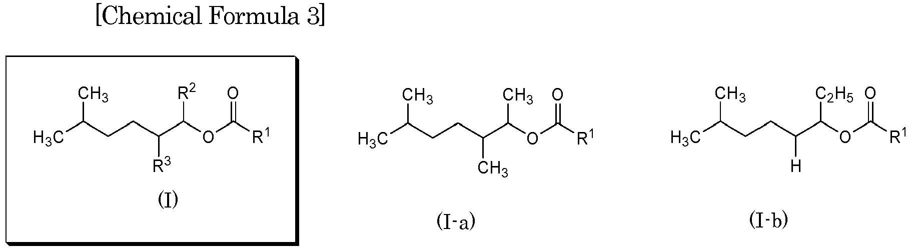

In the formula, R¹ represents methyl, ethyl, propyl, or isopropyl, R² represents methyl or ethyl, and R³ represents methyl or hydrogen, with the proviso that when R² is methyl, R³ is methyl and with the proviso that when R² is ethyl, R³ is hydrogen.

In the formula (I), the formula (I-a), and the formula (I-b), R¹ represents methyl, ethyl, propyl, or isopropyl. In terms of a good fresh and fruity fragrance note, R¹ is preferably methyl, ethyl, or isopropyl, more preferably methyl or ethyl, and further preferably methyl.

In terms of a good fresh and fruity fragrance note, it is preferable that R² is methyl and R³ is methyl in the formula (I). That is, the compound (I-a) is preferred.

In terms of a good fresh and fruity fragrance note, it is preferable that R² is methyl, R³ is methyl, and R¹ is methyl, ethyl, or isopropyl in the formula (I). It is more preferable that R² is methyl, R³ is methyl, and R¹ is methyl or ethyl in the formula (I). It is further preferable that R² is methyl, R³ is methyl, and R¹ is methyl in the formula (I). In terms of a good fresh and fruity fragrance note, R¹ is preferably methyl, ethyl, or isopropyl, more preferably methyl or ethyl, and further preferably methyl in the formula (I-a).

In terms of a good fresh and fruity fragrance note, it is preferable that R² is ethyl, R³ is hydrogen, and R¹ is methyl, ethyl, or isopropyl in the formula (I). It is more preferable that R² is ethyl, R³ is hydrogen, and R¹ is methyl or ethyl in the formula (I). It is further preferable that R² is ethyl, R³ is hydrogen, and R¹ is methyl in the formula (I). In terms of a good fresh and fruity fragrance note, R¹ is preferably methyl, ethyl, or isopropyl, more preferably methyl or ethyl, and further preferably methyl in the formula (I-b).

In the present invention, the ester (I-a), i.e., the ester of the formula (I) in which R² is methyl and R³ is methyl and the ester (I-b), i.e., the ester of the formula (I) in which R² is ethyl and R³ is hydrogen are combined to form a mixture. In terms of a good fresh and fruity fragrance note, the ratio (mass ratio) of the ester (I-a) to the ester (I-b) (ester (I-a) / ester (I-b)) of the mixture is preferably 70/30 to 99.9/0.1, more preferably 80/20 to 99/1, even more preferably 80/20 to 98/2, still more preferably 80/20 to 95/5, and further preferably 90/10 to 95/5.

### [Method for producing ester]

The ester of the formula (I) is obtained by esterification of an alcohol represented by the formula (III) with a carboxylic acid compound.

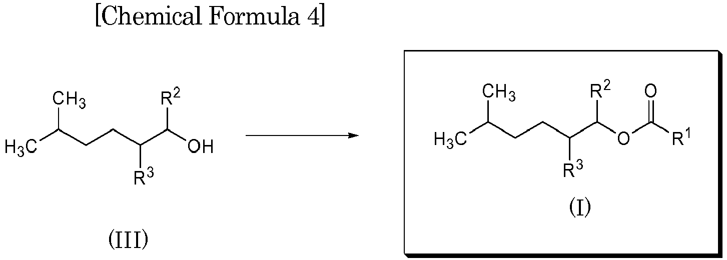

In the formula, R¹ represents methyl, ethyl, propyl, or isopropyl, R² represents methyl or ethyl, and R³ represents methyl or hydrogen, with the proviso that when R² is methyl, R³ is methyl and with the proviso that when R² is ethyl, R³ is hydrogen.

The alcohol of the formula (III) as an alcohol material includes 3,6-dimethylheptane-2-ol (III-a) and 7-methyloctane-3-ol (III-b). The 3,6-dimethylheptane-2-ol may be referred to as "3,6-dimethylheptane-2-ol (III-a)" or a "compound (III-a)" in the present specification. The 7-methyloctane-3-ol represented by the formula (III-b) may be referred to as "7-methyloctane-3-ol (III-b)" or a "compound (III-b)" in the present specification. The compound (III-a) and the compound (III-b) can be obtained by, e.g., the following method.

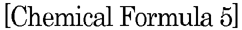

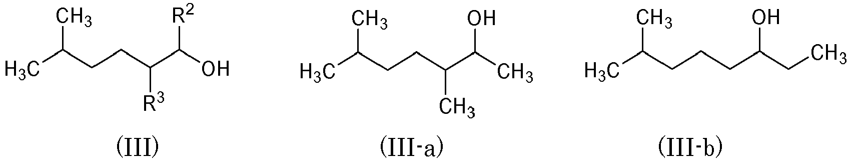

In the formula, R² represents methyl or ethyl and R³ represents methyl or hydrogen, with the proviso that when R² is methyl, R³ is methyl and with the proviso that when R² is ethyl, R³ is hydrogen.

### [Method for producing 3,6-dimethylheptane-2-ol (III-a) and 7-methyloctane-3-ol (III-b)]

The 3,6-dimethylheptane-2-ol (III-a) can be synthesized by a common organic chemical reaction, and the production method is not limited. For example, the compound (III-a) can be produced by a method that includes the following: performing a cross-aldol reaction between isovaleraldehyde (which is represented by the formula (XI) below, and may be referred to as a compound (XI) in the present specification) and 2-butanone (which is represented by the formula (X) below, and may be referred to as a compound (X) in the present specification); then dehydrating the reaction product to form a compound represented by the formula (IV); and reducing the compound represented by the formula (IV) so that the compound (III-a) is produced (see Scheme 1).

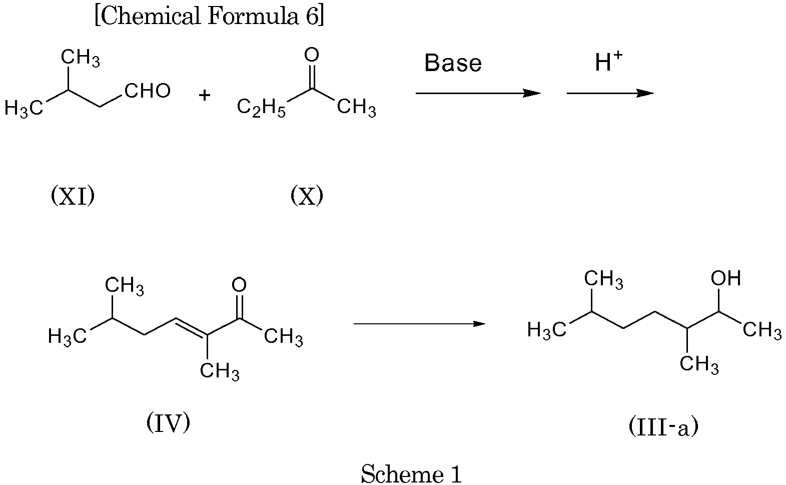

In the above production method, first, the cross-aldol reaction between isovaleraldehyde (XI) and 2-butanone (X) is performed in the presence of a base catalyst. The base catalyst is preferably an alkali metal hydroxide, and more preferably a sodium hydroxide. The reaction is performed, e.g., at 15 to 30°C for 1 to 60 hours.

In particular, it is preferable that water is further added to the reaction solution in order to allow the cross-aldol reaction to proceed with a high yield.

Next, the reaction product is dehydrated. In this case, it is preferable that a phosphoric acid or the like is added to the reaction solution, and the dehydration is performed at a temperature not lower than the boiling point of water. When the pressure of the reaction system is changed, the dehydration is preferably performed at a temperature not lower than the boiling point of water under such a pressure. Moreover, it is preferable that the reaction product is dehydrated by heating, e.g., at 110 to 160°C under a pressure of one atmosphere (101 kPa).

The dehydration results in the formation of 3,6-dimethylhept-3-en-2-one (which is represented by the formula (IV) above, and may be referred to as a compound (IV) in the present specification).

In the cross-aldol reaction, when 7-methyloct-4-en-3-one (which is represented by the formula (V) below, and may be referred to as a compound (V) in the present specification) is obtained together with the compound (IV) (see Scheme 2), the compound (IV) may be separated from the compound (V) by precision distillation or the combination of a silica gel column and distillation. This separation can provide the compound (IV) with a high purity. Alternatively, the conditions of the separation can be adjusted so as to provide a mixture of the compound (IV) and the compound (V) at a specific ratio.

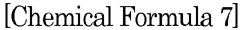

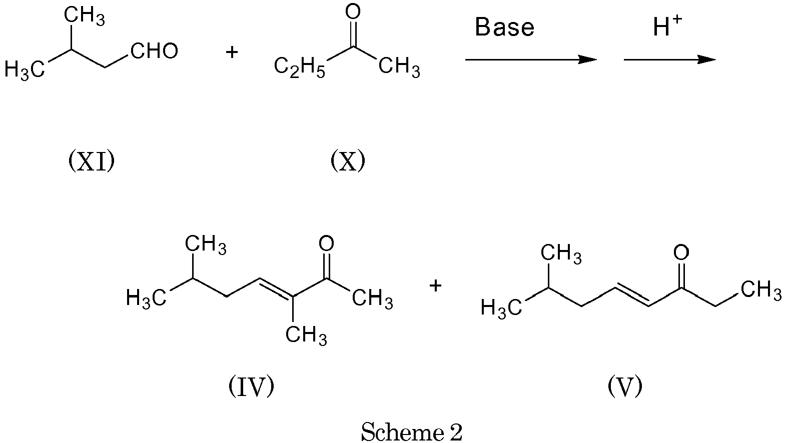

Next, the compound (IV) is reduced to obtain the compound (III-a). Any general reduction method may be used, and a hydrogenation method using a noble metal catalyst is preferred in terms of improving the purity. Specifically, the reduction can be performed by hydrogenation in a hydrogen atmosphere in the presence of a noble metal catalyst such as ruthenium, palladium, or platinum. The hydrogenation is performed under a hydrogen pressure of, e.g., 0.1 to 5 MPa preferably at a temperature of 50 to 170°C, more preferably at a temperature of 60 to 160°C, and further preferably at a temperature of 80 to 160°C. The reaction time for the hydrogenation is, e.g., 3 to 80 hours. Thus, the compound (III-a) with a high purity can be obtained.

On the other hand, when the compound (IV) is not separated from the compound (V), and the mixture of these compounds is reduced, a mixture of the compound (III-a) and 7-methyloctane-3-ol (III-b) can be obtained (see Scheme 3).

In order to obtain the compound (III-a) and the compound (III-b) individually, they may be separated from each other by column chromatography.

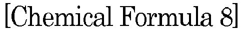

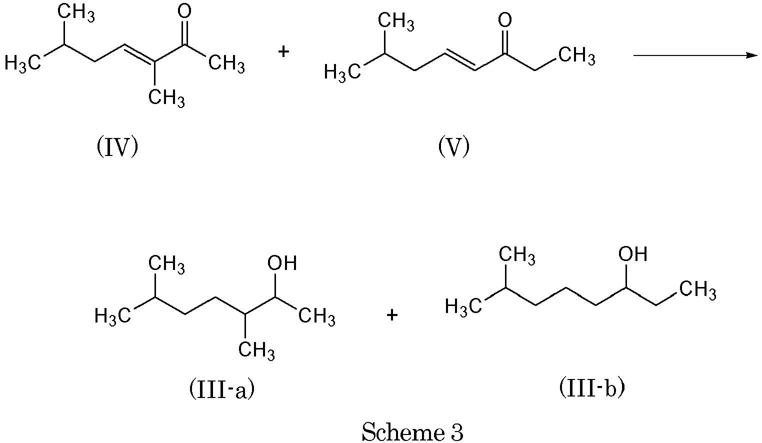

### [Esterification process]

The ester (I) is obtained by esterification of the compound (III), which is produced by the above method, with a carboxylic acid compound that includes an alkyl group having 1 to 3 carbon atoms.

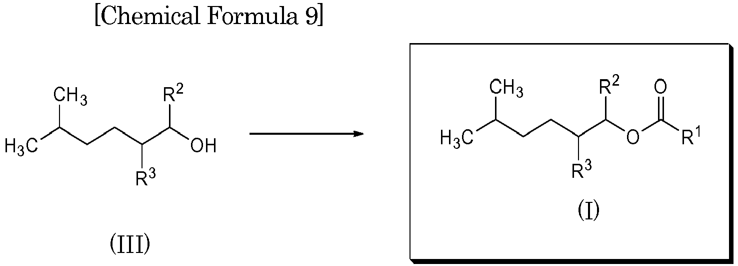

In the formula, R¹ represents methyl, ethyl, propyl, or isopropyl, R² represents methyl or ethyl, and R³ represents methyl or hydrogen, with the proviso that when R² is methyl, R³ is methyl and with the proviso that when R² is ethyl, R³ is hydrogen.

The carboxylic acid compound means a carboxylic acid, an anhydride of a carboxylic acid, or an ester of a carboxylic acid and an alcohol. The carboxylic acid compound is preferably a carboxylic acid that includes an alkyl group having 1 to 3 carbon atoms, an anhydride of a carboxylic acid that includes an alkyl group having 1 to 3 carbon atoms, or an ester of a carboxylic acid that includes an alkyl group having 1 to 3 carbon atoms and an alcohol. Among them, in terms of the reactivity, the carboxylic acid that includes an alkyl group having 1 to 3 carbon atoms, and the anhydride of the carboxylic acid that includes an alkyl group having 1 to 3 carbon atoms are preferred, and the anhydride of the carboxylic acid that includes an alkyl group having 1 to 3 carbon atoms is more preferred.

Any general esterification method may be used, and it is preferable that the compound (I) is obtained by esterification of the compound (III) with the anhydride of a carboxylic acid represented by the formula (II).

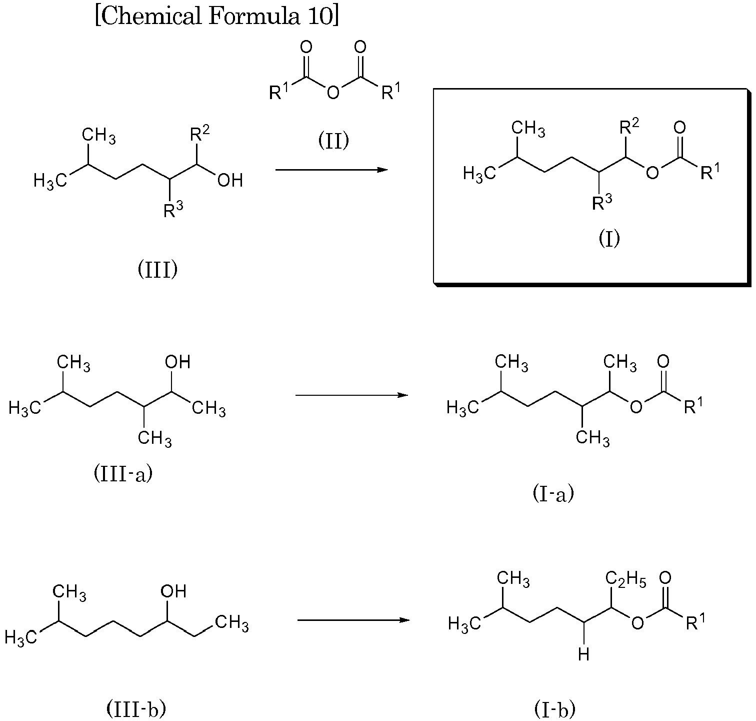

In terms of improving the reactivity, it is more preferable that dimethylaminopyridine (DMAP) is used as a catalyst in the esterification. For reaction control, it is more preferable that the reaction temperature of the esterification is an ice-cold temperature in the early stage of the reaction. After no heat generation is observed during the esterification reaction, the temperature is preferably increased to 15 to 30°C. The reaction time for the esterification is preferably 1 to 10 hours.

In the esterification process, when the mixture of the compound (III-a) and the compound (III-b) is used as a material, the resultant ester may be separated into the compound (I-a) and the compound (I-b) by precision distillation or the combination of a silica gel column and distillation. This separation can provide both the compound (I-a) and the compound (I-b) with a high purity. Alternatively, the conditions of the separation can be adjusted so as to provide a mixture of the compound (I-a) and the compound (I-b) at a specific ratio.

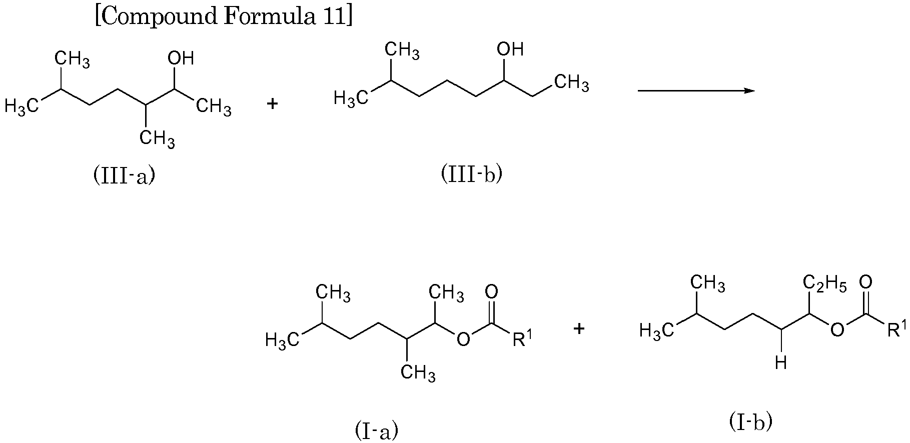

### [Fragrance composition]

As described above, the fragrance composition of the present invention contains the compound (I-a). The content of the compound (I-a) in the fragrance composition is preferably 0.01 to 100 % by mass, more preferably 0.01 to 99.9 % by mass, even more preferably 0.01 to 99 % by mass, still more preferably 0.1 to 15 % by mass, and further preferably 0.3 to 5 % by mass. The fragrance composition containing 0.01 to 100 % by mass of the compound (I-a) can have a good fresh and fruity flavor.

The fragrance composition of the present invention may contain a mixture of the compound (I-a) and the compound (I-b) as the compound (I). The total content of the compound (I-a) and the compound (I-b) in the fragrance composition is preferably 0.01 to 100 % by mass, more preferably 0.01 to 99.9 % by mass, even more preferably 0.01 to 99 % by mass, still more preferably 0.1 to 15 % by mass, and further preferably 0.3 to 5 % by mass. The fragrance composition containing 0.01 to 100 % by mass of the compound (I-a) and the compound (I-b) in total can have a good fresh and fruity flavor.

When the fragrance composition of the present invention contains the mixture of the compound (I-a) and the compound (I-b) as the compound (I), the ratio (mass ratio) of the compound (I-a) to the compound (I-b) (compound (I-a) / compound (I-b)) is preferably 70/30 to 99.9/0.1, more preferably 80/20 to 99/1, and further preferably 90/10 to 97/3.

Moreover, a fabric treatment composition such as a softener, a cleaner composition, or a cosmetic composition such as hair cosmetics of the present invention may contain both the compound (I-a) and the compound (I-b). In this case, the ratio (mass ratio) of the compound (I-a) to the compound (I-b) (compound (I-a) / compound (I-b)) is preferably 70/30 to 99.9/0.1, more preferably 80/20 to 99/1, and further preferably 90/10 to 97/3.

The fragrance composition of the present invention can contain, as a fragrance other than the compound (I), other fragrance components in common use or formulated perfumes with a desired composition. The presence of the fragrances other than the compound (I) can provide the fragrance composition of the present invention with aromas of, e.g., a floral tone, a bouquet tone, a hyacinth tone, a geranium tone, a rose tone, a bergamot tone, an orchid tone, or a lily of the valley tone (muguet).

In the fragrance composition of the present invention, other fragrances that can be used in combination with the compound (I) may include esters other than the compound (I), alcohols, hydrocarbons, phenols, carbonates, aldehydes, ketones, acetals, ethers, carboxylic acids, lactones, nitriles, Schiff bases, and fragrance components such as natural essential oils and natural extracts.

Among them, the esters other than the compound (I), the alcohols, the carbonates, the aldehydes, the ketones, the ethers, and the lactones are preferred. In particular, the esters other than the compound (I) and the alcohols are more preferred.

The alcohols may include terpene alcohols, aromatic alcohols, and aliphatic alcohols. Among them, the terpene alcohols and the aromatic alcohols are preferred.

Examples of the terpene alcohols include linalool, ethyl linalool, citronellol, geraniol, nerol, terpineol, α-terpineol, dihydromyrcenol, farnesol, nerolidol, cedrol, menthol, borneol, and isobornyl cyclohexanol.

Examples of the aromatic alcohols include phenylethyl alcohol, benzyl alcohol, dimethyl benzyl carbinol, phenylethyl dimethyl carbinol, and phenyl hexanol.

Examples of the aliphatic alcohols include cis-3-hexenol, 1-(2,2,6-trimethylcyclohexyl)-3-hexanol, AMBER CORE (Trade Name of Kao Corporation: 1-(2-tert-butylcyclohexyloxy)-2-butanol), SANDALMYSORE CORE (Trade Name of Kao Corporation, 2-methyl-4-(2,2,3-trimethyl-3-cyclopenten-1-yl)-2-buten-1-ol), MAGNOL (Trade Name of Kao Corporation, a mixture containing ethyl norbornyl cyclohexanol as a main component), and UNDECAVERTOL (Trade Name of Givaudan: 4-methyl-3-decene-5-ol).

The hydrocarbons may include limonene, α-pinene, β-pinene, terpinene, cedrene, longifolene, and valencene.

The phenols may include guaiacol, eugenol, isoeugenol, thymol, p-cresol, vanillin, and ethyl vanillin.

The esters other than the compound (I) may include formate ester, acetate ester, propionate ester, butyrate ester, valerate ester, hexanoate ester, heptanoate ester, nonenoate ester, phenylacetate ester, benzoate ester, cinnamate ester, salicylate ester, brassylate ester, tiglate ester, jasmonate ester, dihydrojasmonate ester, glycidic ester, and anthranilate ester.

Among these esters, the acetate ester, the propionate ester, the salicylate ester, and the dihydrojasmonate ester are preferably used.

Examples of the formate ester include linalyl formate, citronellyl formate, and geranyl formate.

Examples of the acetate ester include hexyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, benzyl acetate, phenylethyl acetate, styralyl acetate, cinnamyl acetate, dimethylbenzylcarbinyl acetate, and 3-pentyltetrahydropyran-4-yl acetate.

Examples of the propionate ester include citronellyl propionate, tricyclodecenyl propionate, allylcyclohexyl propionate, benzyl propionate, and styralyl propionate.

Examples of the butyrate ester include citronellyl butyrate, dimethylbenzylcarbinyl n-butyrate, and tricyclodecenyl isobutyrate.

Examples of the valerate ester include methyl valerate, ethyl valerate, butyl valerate, amyl valerate, benzyl valerate, and phenylethyl valerate. Examples of the hexanoate ester include methyl hexanoate, ethyl hexanoate, allyl hexanoate, linalyl hexanoate, and citronellyl hexanoate.

Examples of the heptanoate ester include methyl heptanoate and allyl heptanoate.

Examples of the nonenoate ester include methyl 2-nonenoate, ethyl 2-nonenoate, and ethyl 3-nonenoate. Examples of the phenylacetate ester include phenylethyl phenyl acetate and p-cresyl phenylacetate.

Examples of the benzoate ester include methyl benzoate, benzyl benzoate, and methyl 2,4-dihydroxy-3,6-dimethylbenzoate.

Examples of the cinnamate ester include methyl cinnamate and benzyl cinnamate.

Examples of the salicylate ester include methyl salicylate, n-hexyl salicylate, cis-3-hexenyl salicylate, cyclohexyl salicylate, and benzyl salicylate.

Examples of the brassylate ester include ethylene brassylate.

Examples of the tiglate ester include geranyl tiglate, 1-hexyl tiglate, and cis-3-hexenyl tiglate.

Examples of the jasmonate ester include methyl jasmonate.

Examples of the dihydrojasmonate ester include methyl dihydrojasmonate.

Examples of the anthranilate ester include methyl anthranilate, ethyl anthranilate, and dimethyl anthranilate (methyl N-methyl anthranilate).

Examples of the other esters include ethyl 2-methyl butyrate, methyl atrarate, allyl cyclohexyl glycolate, FRUITATE (Trade Name of Kao Corporation: ethyl tricyclo[5.2.1.0^{2,6}]decan-2-carboxylate), POIRENATE (Trade Name of Kao Corporation: ethyl 2-cyclohexyl propionate), PERANAT (Trade Name of Kao Corporation: 2-methylpentyl 2-methylvalerate), MELUSAT (Trade Name of Kao Corporation: ethyl 3,5,5-trimethyl hexanoate), and IROTYL (Trade Name of Kao Corporation, ethyl 2-ethylcapronate).

The carbonates may include LIFFAROME (Trade Name of IFF: cis-3-hexenyl methyl carbonate), JASMACYCLAT (Trade Name of Kao Corporation: methyl cyclooctyl carbonate), and FLORAMAT (Trade Name of Kao Corporation, ethyl 2-tert-butylcyclohexyl carbonate).

The aldehydes may include n-octanal, n-nonanal, n-decanal, n-dodecanal, 2-methyl undecanal, 10-undecenal, citronellal, citral, hydroxycitronellal, TRIPLAL (Trade Name of IFF: 2,4-dimethyl-3-cyclohexene-1-carboxaldehyde), CYCLOVERTAL (Trade Name of Kao Corporation: dimethyl-3-cyclohexenyl-1-carboxaldehyde), benzaldehyde, phenylacetaldehyde, phenylpropylaldehyde, cinnamaldehyde, dimethyltetrahydrobenzaldehyde, BOURGEONAL (Trade Name of Givaudan: 3-(p-tert-butylphenyl)-propanal), LYRAL (Trade Name of IFF: hydroxy myrac aldehyde), POLLENAL II (Trade Name of Kao Corporation: 2-cyclohexyl propanal), LILIAL (Trade Name of Givaudan: p-tert-butyl-α-methyl hydrocinnamaldehyde), p-isopropyl-α-methyl hydrocinnamaldehyde, FLORALOZONE (Trade Name of IFF: p-ethyl-α,α-dimethyl hydrocinnamaldehyde), α-amyl cinnamaldehyde, α-hexyl cinnamaldehyde, heliotropin, and HELIONAL (Trade Name of IFF: α-methyl-3,4-methylenedioxy hydrocinnamaldehyde).

The ketones may include α-ionone, β-ionone, γ-ionone, α-methyl ionone, β-methyl ionone, γ-methyl ionone, damascenone, methyl heptenone, 4-methylene-3,5,6,6-tetramethyl-2-heptanone, acetophenone, amyl cyclopentanone, dihydrojasmone, rose ketone, carvone, menthone, camphor, acetyl cedrene, isolongifolanone, nootkatone, benzylacetone, anisylacetone, methyl β-naphthyl ketone, 2,5-dimethyl-4-hydroxy-3(2H)-furanone, maltol, muscone, civetone, cyclopentadecanone, CALONE (Trade Name of Firmenich:
7-methyl-3,4-dihydro-2H-benzodioxepin-3-one), raspberry ketone, and heliotropyl acetone.

The acetals may include acetaldehyde ethylphenylpropyl acetal, citral diethyl acetal, phenylacetaldehyde glyceryl acetal, phenylacetaldehyde dimethyl acetal, ethyl acetoacetate ethylene glycol acetal, BOISAMBRENE FORTE (Trade Name of Kao Corporation), and TROENAN (Trade Name of Kao Corporation).

The ethers may include cedryl methyl ether, estragole, anethole, β-naphthyl methyl ether, β-naphthyl ethyl ether, limonene oxide, rose oxide, nerol oxide, 1,8-cineole, rose furan, AMBROXAN (Trade Name of Kao Corporation: dodecahydro-3a,6,6,9a-tetramethyl naphtho[2.1-b]furan), HERBAVERT (Trade Name of Kao Corporation: 3,3,5-trimethylcyclohexyl ethyl ether), and GALAXOLIDE (Trade Name of IFF: hexamethylhexahydrocyclopentabenzopyran).

The carboxylic acids may include benzoic acid, phenylacetic acid, cinnamic acid, hydrocinnamic acid, butyric acid, and 2-hexenoic acid.

The lactones may include γ-decalactone, δ-decalactone, γ-valerolactone, γ-nonalactone, γ-undecalactone, δ-hexalactone, γ-jasmolactone, whisky lactone, coumarin, cyclopentadecanolide, cyclohexadecanolide, ambrettolide, 11-oxahexadecanolide, and butylidenephthalide. The nitriles may include geranyl nitrile, citronellyl nitrile, and dodecanenitrile.

The Schiff bases may include aurantiol and ligantral. The natural essential oils and natural extracts may include orange, lemon, lime, bergamot, vanilla, mandarin, peppermint, spearmint, lavender, chamomile, rosemary, eucalyptus, sage, basil, rose, rockrose, geranium, jasmine, ylang ylang, anise, clove, ginger, nutmeg, cardamon, cedar, cypress, vetiver, patchouli, lemongrass, labdanum, and grapefruit.

The content of the fragrances other than the compound (I) may be appropriately selected based on, e.g., the type of the formulated perfumes, and the type and intensity of the intended odor. The content of each of these fragrances in the fragrance composition is preferably 0.0001 to 99.99 % by mass, and more preferably 0.001 to 80 % by mass. The total content of the other fragrances in the fragrance composition is preferably 5 to 99.99 % by mass, and more preferably 50 to 99.95 % by mass.

The fragrance composition of the present invention may contain an odorless oil as a base for incorporating the compound (I) and other fragrance materials. The use of such an oil allows the fragrance components to be uniformly mixed together and easily added to a product, and thus makes it easy to impart an odor of moderate intensity to the product. Specific examples of the oil include the following: polyhydric alcohols such as ethylene glycol, propylene glycol, butylene glycol, and dipropylene glycol; esters such as isopropyl myristate, dibutyl adipate, and diethyl sebacate; and hydrocarbons such as liquid paraffin and squalane. The content of the oil in the fragrance composition is preferably 1 to 95 % by mass, more preferably 10 to 80 % by mass, and further preferably 15 to 50 % by mass.

The fragrance composition of the present invention may contain a surfactant such as polyoxyethylene alkyl ether or sorbitan fatty acid ester.

### [Use as fragrance-imparting component]

The compound (I) serves as a fragrance material having a fresh and fruity fragrance note and can be used as a fragrance-imparting component for various products. Moreover, the fragrance composition containing the compound (I) serves as a formulated perfume having a fresh and fruity fragrance note and can be used as a fragrance-imparting component for various products.

Therefore, the present invention is directed to a method for using the fragrance composition containing the compound (I-a) as a fragrance-imparting component (i.e., a fragrance). The compound (I-a) and the fragrance composition containing the compound (I-a) may be used so that they are contained alone or in combination with other components in the bases of toiletry products such as soaps, cosmetics, hair cosmetics, detergents, softeners, spray products, air fresheners, perfume, and bath agents. Accordingly, the present invention is directed to a method for using the fragrance composition containing the compound (I-a) as a fragrance for toiletry products such as soaps, cosmetics, hair cosmetics, detergents, softeners, spray products, air fresheners, perfumes, and bath agents.

In the method for using the fragrance composition containing the compound (I) as a fragrance-imparting component, the total amount of the compound (I-a) and the compound (I-b), which constitute the compound (I), is preferably 0.01 to 100 % by mass, more preferably 0.01 to 99.9 % by mass, even more preferably 0.01 to 99 % by mass, still more preferably 0.1 to 15 % by mass, and further preferably 0.3 to 5 % by mass with respect to the entire fragrance composition. The fragrance composition containing 0.01 to 100 % by mass of the compound (I) can have a fresh and fruity flavor.

In particular, it is preferable that the compound (I-a) and the fragrance composition containing the compound (I-a) of the present invention are used to treat hairs or fabrics of clothing etc. and to give them a fruity aroma. Examples of the products in which the compound (I-a) and the fragrance composition containing the compound (I-a) can be suitably used as fragrance-imparting components include hair cosmetics such as shampoos and hair conditioners, and softeners for clothing.

Therefore, the present invention also provides a cleaner composition containing the fragrance composition of the present invention, a cosmetic composition containing the fragrance composition of the present invention, a fabric treatment composition containing the fragrance composition of the present invention, a fragrant deodorant containing the fragrance composition of the present invention, and a cleaning nonwoven fabric containing the fragrance composition of the present invention.

The cleaner composition of the present invention is preferably a body cleaner composition, a cleaner composition for clothing, or a cleaner composition for hard surfaces, more preferably a body cleaner composition or a cleaner composition for clothing, and further preferably a cleaner composition for clothing. The cleaner composition of the present invention may be a powder cleaner composition or a liquid cleaner composition, and the liquid cleaner composition is preferred.

Examples of the body cleaner composition include a skin cleaner composition and a hair cleaner composition, and the skin cleaner composition is preferred.

Examples of the cleaner composition for hard surfaces include an all purpose cleaner and a dish cleaner composition.

The cleaner composition of the present invention preferably contains an anionic surfactant other than the compound (I) or the fragrance composition containing the compound (I). The cleaner composition may further contain, e.g., a nonionic surfactant, a pH adjuster, a viscosity modifier, a solvent, an oil, an antiseptic agent, or water.

The fabric treatment composition of the present invention is preferably a softener.

The softener of the present invention contains, e.g., quaternary ammonium salts or salts of tertiary amine etc. and the fragrance composition of the present invention. The softener may further contain, e.g., a germicide, a viscosity modifier, a pH adjuster, or a solvent.

Any conventionally known quaternary ammonium salts may be used. Examples of the quaternary ammonium salts include the following: octyl trimethyl ammonium chloride; dodecyl trimethyl ammonium chloride; hexadecyl trimethyl ammonium chloride; beef tallow trimethyl ammonium chloride; coconut oil trimethyl ammonium chloride; octyl dimethyl benzyl ammonium chloride; decyl dimethyl benzyl ammonium chloride; dioctadecyl dimethyl ammonium chloride; distearoyloxyethyl dimethyl ammonium chloride; dioleoyloxyethyl dimethyl ammonium chloride; N-stearoyloxyethyl-N,N-dimethyl-N-(2-hydroxyethyl)ammonium methyl sulfate; N,N-distearoyloxyethyl-N-methyl-N-(2-hydroxyethyl)ammonium methyl sulfate; N-oleoyloxyethyl-N,N-dimethyl-N-(2-hydroxyethyl)ammonium methyl sulfate; methyl-1-beef tallow amide ethyl-2-beef tallow alkyl imidazolinium methyl sulfate; methyl-1-hexadecanoyl amide ethyl-2-pentadecyl imidazolinium chloride; ethyl-1-octadecenoyl amide ethyl-2-heptadecenyl imidazolinium ethyl sulfate; and N,N-di[2-(alkanoyloxy)-ethyl]-N-(2-hydroxyethyl)-N-methyl ammonium sulfate. One of them may be used alone or two or more of them may be used in combination.

Any conventionally known salts of tertiary amine etc. may be used. Examples of the salts of tertiary amine etc. include the following: distearyl methylamine hydrochloride; dioleyl methylamine hydrochloride; distearyl methylamine sulfate;
N-(3-octadecanoylaminopropyl)-N-(2-octadecanoyloxyethyl-N-methylamine hydrochloride; 1-octadecanoylaminoethyl-2-heptadecyl imidazoline hydrochloride; and 1-octadecenoylaminoethyl-2-heptadecenyl imidazoline hydrochloride. One of them may be used alone or two or more of them may be used in combination.

Examples of the germicide include the following: alcohols having 1 to 8 carbon atoms; benzoic acids; and phenols. Specifically, the germicide may be, e.g., ethanol, propylene glycol, benzyl alcohol, salicylic acid, methyl p-hydroxybenzoate, or cresol.

Examples of the viscosity modifier include inorganic or organic salts (excluding the quaternary ammonium salts or the salts of tertiary amine etc.). Specifically, the viscosity modifier may be, e.g., sodium chloride, potassium chloride, calcium chloride, magnesium chloride, aluminum chloride, sodium sulfate, magnesium sulfate, potassium sulfate, sodium nitrate, magnesium nitrate, sodium p-toluenesulfonate, sodium glycolate, sodium acetate, potassium acetate, potassium glycolate, or sodium lactate. Among them, the calcium chloride and the magnesium chloride are preferred.

The softener of the present invention contains water, and the remainder of the composition is generally water. The water is preferably ion exchanged water or distilled water. The pH of the softener of the present invention is preferably 1.5 to 6 at 20°C. In terms of the antiseptic/bactericidal activity, the pH of the softener should be as low as possible. However, if the pH is too low, there is a possibility that the components that are generally contained in the composition will be decomposed. Therefore, the pH is more preferably 1.5 to 5, and further preferably 2 to 4.5.

Any inorganic or organic acid and alkali can be used to adjust the pH of the softener of the present invention.

In addition to the above components, the softener of the present invention may contain as optional components any known components, which are usually mixed in a softener, to the extent that they do not interfere with the effects of the present invention. Examples of the optional components include the following: higher fatty acids such as stearic acid, oleic acid, and palmitic acid or esters of the higher fatty acids and lower alcohols; nonionic surfactants such as fatty acid glycerol ester that is derived from, e.g., stearic acid and glycerin; higher alcohols such as stearyl alcohol, palmityl alcohol, and oleyl alcohol; and low-temperature stabilizers such as ethylene glycol and glycerin. Moreover, the softener may contain, e.g., urea, pigments, cellulose derivatives, an ultraviolet absorber, or a fluorescent brightener.

The cosmetic composition of the present invention is preferably perfume, body cosmetics, hair cosmetics, or a body cream.

The perfume of the present invention may contain, e.g., a solvent or water other than the compound (I-a) or the fragrance composition containing the compound (I-a).

With respect to the above embodiment, the present invention further discloses the following fragrance composition, method for producing the fragrance composition, method for using the fragrance composition, and use of the fragrance composition.
<1> An ester represented by the formula (I). 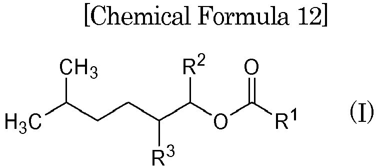
   (In the formula, R¹ represents methyl, ethyl, propyl, or isopropyl, R² represents methyl or ethyl, and R³ represents methyl or hydrogen, with the proviso that when R² is methyl, R³ is methyl and with the proviso that when R² is ethyl, R³ is hydrogen.)
<2> The ester according to item <1>, wherein R² is methyl and R³ is methyl.
<3> The ester according to item <1>, wherein R² is ethyl and R³ is hydrogen.
<4> The ester according to any one of items <1> to <3>, wherein R¹ is preferably methyl, ethyl, or isopropyl, more preferably methyl or ethyl, and further preferably methyl.
<5> A fragrance composition containing the ester according to any one of items <1> to <4>.
<6> The fragrance composition according to item <5>, wherein a content of the ester according to any one of items <1> to <4> in the fragrance composition is preferably 0.01 to 100 % by mass, more preferably 0.01 to 99.9 % by mass, even more preferably 0.01 to 99 % by mass, still more preferably 0.1 to 15 % by mass, and further preferably 0.3 to 5 % by mass.
<7> A fragrance composition containing the ester according to item <1> in which R² is methyl and R³ is methyl, and the ester according to item <1> in which R² is ethyl and R³ is hydrogen.
<8> The fragrance composition according to item <6>, wherein a mass ratio of the ester according to item <1> in which R² is methyl and R³ is methyl to the ester according to item <1> in which R² is ethyl and R³ is hydrogen (the ester according to item <1> in which R² is methyl and R³ is methyl / the ester according to item <1> in which R² is ethyl and R³ is hydrogen) is preferably 70/30 to 99.9/0.1, more preferably 80/20 to 99/1, and further preferably 90/10 to 97/3.
<9> The fragrance composition according to item <7> or <8>, wherein a total content of the ester according to item <1> in which R² is methyl and R³ is methyl and the ester according to item <1> in which R² is ethyl and R³ is hydrogen in the fragrance composition is preferably 0.01 to 100 % by mass, more preferably 0.01 to 99.9 % by mass, even more preferably 0.01 to 99 % by mass, still more preferably 0.1 to 15 % by mass, and further preferably 0.3 to 5 % by mass.
<10> The fragrance composition according to any one of items <5> to <9>, further containing at least one selected from the group consisting of esters other than the ester represented by the formula (I), alcohols, carbonates, aldehydes, ketones, ethers, and lactones.
<11> The fragrance composition according to any one of items <5> to <10>, further containing an oil.
<12> The fragrance composition according to any one of items <5> to <11>, further containing a surfactant (e.g., polyoxyethylene alkyl ether or sorbitan fatty acid ester).
<13> A cleaner composition containing the fragrance composition according to any one of items <5> to <12>.
<14> A cleaner composition for clothing containing the fragrance composition according to any one of items <5> to <12>.
<15> A fabric treatment composition containing the fragrance composition according to any one of items <5> to <12>.
<16> A softener containing the fragrance composition according to any one of items <5> to <12>.
<17> A cosmetic composition containing the fragrance composition according to any one of items <5> to <12>.
<18> Perfume, body cosmetics, or hair cosmetics containing the fragrance composition according to any one of items <5> to <12>.
<19> A body cream containing the fragrance composition according to any one of items <5> to <12>.
<20> A cleaner composition (preferably a body cleaner composition (e.g., a skin cleaner composition or a hair cleaner composition), a cleaner composition for clothing, or a cleaner composition for hard surfaces (e.g., an all purpose cleaner or a dish cleaner composition), more preferably a body cleaner composition or a cleaner composition for clothing, and further preferably a cleaner composition for clothing) containing the fragrance composition according to any one of items <5> to <12>.
<21> A method for using the ester according to any one of items <1> to <4> as a fragrance-imparting component.
<22> A method for producing an ester represented by the formula (I), including esterification of an alcohol represented by the formula (III) with a carboxylic anhydride represented by the formula (II). 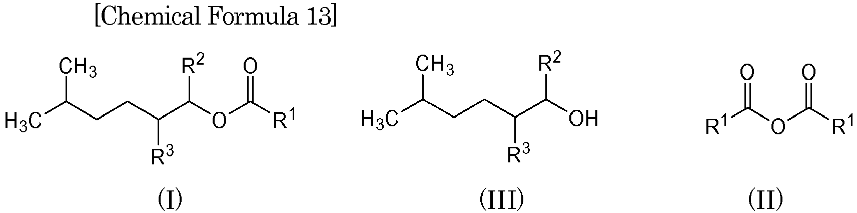
   (In the formula, R¹ represents methyl, ethyl, propyl, or isopropyl, R² represents methyl or ethyl, and R³ represents methyl or hydrogen, with the proviso that when R² is methyl, R³ is methyl and with the proviso that when R² is ethyl, R³ is hydrogen.)
<23> The method for producing an ester according to item <22>, wherein the alcohol represented by the formula (III) in which R² is methyl and R³ is methyl (i.e., the alcohol represented by the formula (III-a)) is obtained by reducing a compound represented by the formula (IV). 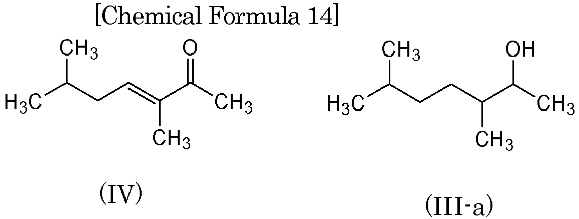
<24> The method for producing an ester according to item <23>, wherein the compound represented by the formula (IV) is obtained by performing a cross-aldol reaction between isovaleraldehyde represented by the formula (XI) and 2-butanone represented by the formula (X), and then dehydrating the reaction product. 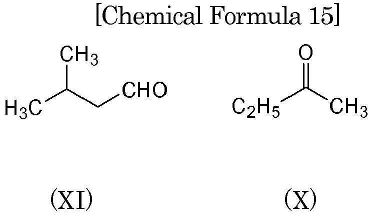
<25> The method for producing an ester according to item <22>, wherein the alcohol represented by the formula (III) in which R² is methyl and R³ is methyl (i.e., the alcohol represented by the formula (III-a)) is separated from a mixture that is obtained by reducing the compound represented by the formula (IV) and a compound represented by the formula (V). 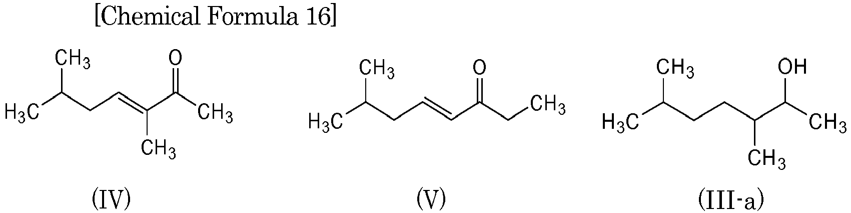
<26> The method for producing an ester according to item <22>, wherein the alcohol represented by the formula (III) in which R² is ethyl and R³ is hydrogen (i.e., the alcohol represented by the formula (III-b)) is separated from a mixture that is obtained by reducing the compound represented by the formula (IV) and the compound represented by the formula (V). 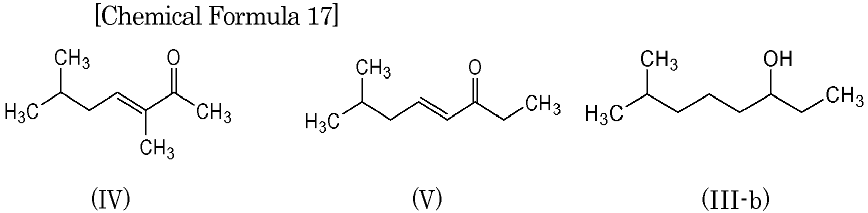
<27> Use of the ester according to any one of items <1> to <4> as a fragrance-imparting component.

### [Examples]

Details of the measurement methods performed in the following production examples or the like will be described together below. In the following, the compounds prepared in Examples 1-4 (I-a-1 to I-a-4) are esters of the present invention whereas the compounds prepared in Examples 5-8 (I-b-1 to I-b-4) are esters that are described for reference purposes.

### [Compound Identification]

The structure of each compound obtained in the following production examples or the like was identified by a nuclear magnetic resonance spectrum (¹H-NMR, ¹³C-NMR). The nuclear magnetic resonance spectrum was measured by "Mercury 400" (product name) manufactured by Varian with the use of chloroform-d as a solvent.

### [Odor Evaluation]

One expert, who had an experience of seven years of perfume blending and fragrance evaluation, determined the fragrance note by a smelling strip method. In the smelling strip method, the tip of a smelling strip (fragrance test paper with a width of 6 mm and a length of 150 mm) was immersed about 5 mm in a sample, and thus the sample was evaluated.

For comparison of odors, the odors that were primarily perceived were listed in the order of their strength, and the freshness was also assessed in accordance with the following criteria. Table 1 shows the results.

### < Criteria for assessment >

4: Freshness was strongly felt.
3: Freshness was felt.
2: Freshness was weakly felt.
1: Freshness was not felt.

### [Production Example 1]

Production of 3,6-dimethylhept-3-en-2-one (compound (IV)) and 7-methyloct-4-en-3-one (Compound (V))

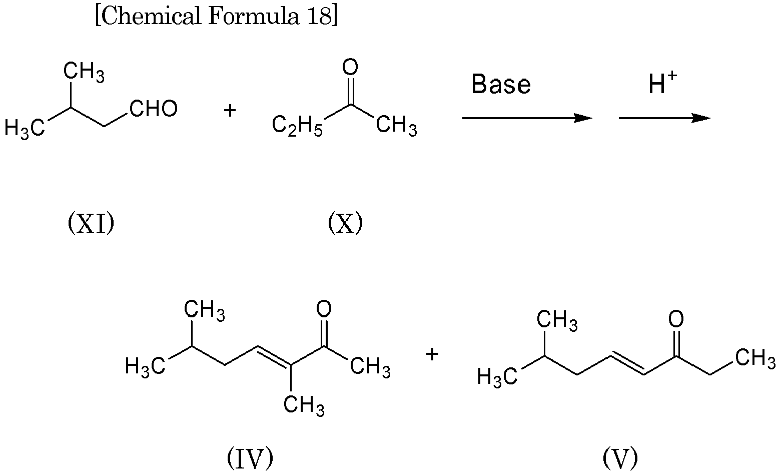

Sodium hydroxide (0.2 g), water (40 g), and 2-butanone (X) (84 g, 1.16 mol) were placed in a flask and maintained at 15°C. Then, isovaleraldehyde (XI) (40 g, 0.46 mol) was dropped into the flask for 5 hours while stirring. Moreover, sodium hydroxide (0.4 g) was further added, and the mixture was further stirred at 25°C for 48 hours. After stirring, the reaction solution was allowed to stand still, and the lower layer was removed. Thereafter, excess 2-butanone (X) was distilled off from the upper layer.

Subsequently, 85% phosphoric acid (2 g) was added to the upper layer portion of the reaction solution. A water fractionator was attached to the flask, and the reaction solution was heated to 120°C and dehydrated. Sodium hydroxide (1.4 g) was added to the reaction solution after the dehydration so that the reaction solution was neutralized. Then, the reaction solution was dried by the addition of magnesium sulfate. Thereafter, the magnesium sulfate was removed by filtration. The reaction solution thus obtained was concentrated to provide a residue (42 g).

The residue was purified by a silica gel column (using hexane : ethyl acetate = 99 : 1 (v/v) as eluent). The purified product was further purified by distillation, so that 22 g of compound (IV) and 4 g of compound (V) were obtained.

The compound (IV) had a purity of 97% and the compound (V) had a purity of 94%.

### [Production Example 2]

Production of 3,6-dimethylheptane-2-ol (compound (III-a))

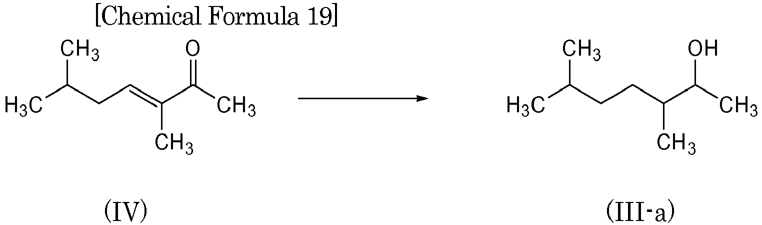

5% active carbon supporting ruthenium catalyst (0.6 g), isopropyl alcohol (5.0 g), and the compound (IV) (12 g) produced in Production Example 1 were placed in a pressure resistant vessel and hydrogenated under a hydrogen pressure of 0.5 MPa at 90°C for 6.5 hours. The reaction solution thus obtained was filtered, and 12 g of filtrate was obtained (with a yield of 95%).

The filtrate was concentrated, and then the concentrated solution was purified by a silica gel column (using hexane : ethyl acetate = 97 : 3 (v/v) as eluent). The purified product was further purified by distillation, so that 10 g of compound (III-a) was obtained. The compound (III-a) had a purity of 100%.

The following is the results of ¹H-NMR, and ¹³C-NMR measurements of the compound (III-a).
¹H-NMR (CDCl₃, 400 MHz, δ ppm): 0.85-0.92 (m, 9H), 1.02-1.20 (m, 5H), 1.20-1.36 (m, 2H), 1.36-1.58 (m, 3H), 3.62-3.76 (m, 1H)
¹³C-NMR (CDCl₃, 400 MHz, δ ppm): 14.7 (CH₃), 15.0 (CH₃), 19.7 (CH₃), 20.7 (CH₃), 22.9 (CH₃), 23.3 (CH₃), 28.7 (CH₂), 30.7 (CH), 30.8 (CH), 37.0 (CH₂), 37.0 (CH₂), 40.4 (CH), 40.7 (CH), 71.7 (CH), 72.0 (CH)

### [Production Example 3]

### Production of mixture of compound (III-a) and compound (III-b)

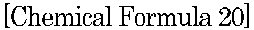

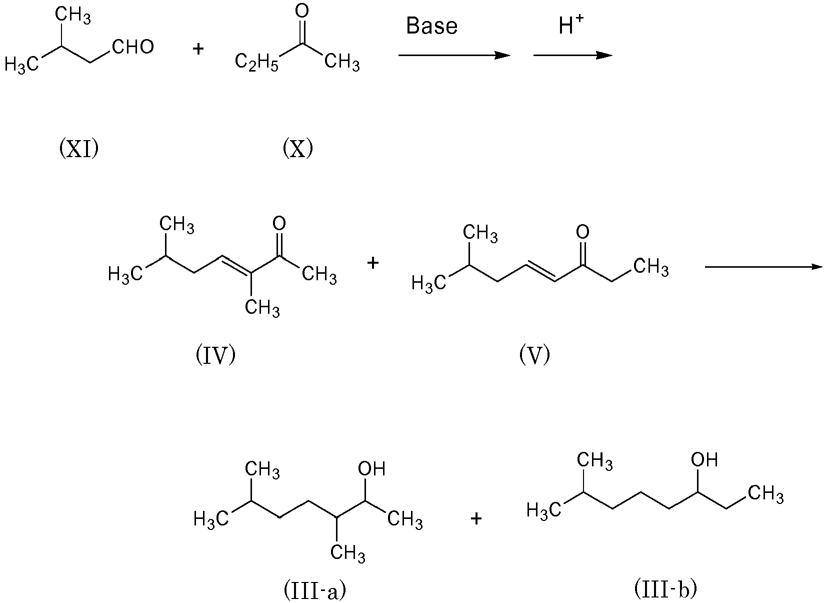

Sodium hydroxide (16 g), water (1.1 kg), and 2-butanone (X) (2.3 kg, 33 mol) were placed in a flask, and then isovaleraldehyde (XI) (1.1 kg, 14 mol) was dropped into the flask at a normal temperature of 26°C for 3 hours while stirring. Moreover, sodium hydroxide (9.6 g) was further added, and the mixture was further stirred at 26°C for 20 hours. After stirring, the reaction solution was allowed to stand still, and the lower layer was removed. Thereafter, 82 g of 6N aqueous sulfuric acid solution was added to neutralize the upper layer, and then excess 2-butanone (X) was distilled off from the upper layer.

Subsequently, 85% phosphoric acid (48 g) was added to the upper layer portion of the reaction solution. A water fractionator was attached to the flask, and the reaction solution was heated to 120°C under a reduced pressure of 6.7 kPa and dehydrated. Sodium hydroxide (30 g) was added to the reaction solution after the dehydration so that the reaction solution was neutralized. Then, the reaction solution was dried by the addition of magnesium sulfate. Thereafter, the magnesium sulfate was removed by filtration. The reaction solution thus obtained was concentrated to provide a residue (1.6 kg).

The residue was subjected to distillation under reduced pressure (up to 130°C / up to 5.0 kPa), thereby providing a fraction with a composition in which the mass ratio of the compound (IV) to the compound (V) (compound (IV): compound (V)) was 92 : 8.

5% active carbon supporting ruthenium catalyst (42 g) and the fraction (1.4 kg) were placed in a pressure resistant vessel and hydrogenated under a hydrogen pressure of 0.6 MPa at 98°C for 53 hours. The reaction solution thus obtained was filtered, and 1.4 kg of filtrate was obtained (with a yield of 99%).

1.3 kg of the filtrate (in which the mass ratio of the compound (III-a) to the compound (III-b) (compound (III-a) : compound (III-b)) was 91: 9) was distilled with a 20-step rectifying column (the initial distillation: 112°C / 2.6 kPa, a reflux ratio of 20; the main distillation: 112 to 125°C / 2.6 to 1.3 kPa, a reflux ratio of 10 to 5; the post-distillation: 125 to 150°C / 1.3 to 0.8 kPa, a reflux ratio of 5; and the final distillation: 150°C / 0.8 kPa, a reflux ratio of 5). In the main distillation, a fraction (1.1 kg) with a mass ratio of 95 : 5 (compound (III-a): compound (III-b)) was obtained. The main distillation time was 6.8 hours. In the post-distillation, a fraction (21 g) with a mass ratio of 63 : 37 (compound (III-a) : compound (III-b)) was obtained. The post distillation time was 1.4 hours.

### [Example 1]

### Production of 3,6-dimethylheptane-2-yl acetate (compound (I-a-1))

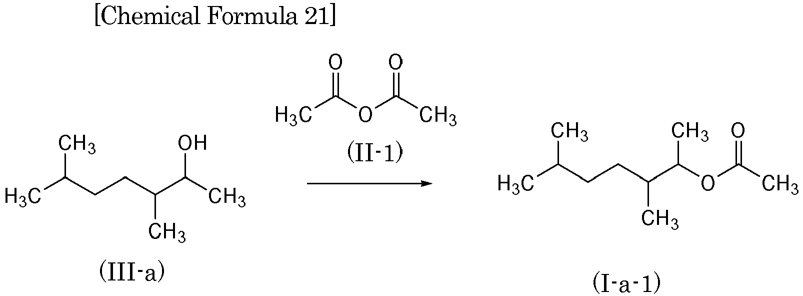

3,6-dimethylheptane-2-ol (compound (III-a)) (5.0 g, 34 mmol) produced in Production Example 2 and acetic anhydride (II-1) (5.0 g, 42 mmol) were placed in a flask and cooled in an ice bath. Then, dimethylaminopyridine (referred to as DMAP in the following) (53 mg, 0.4 mmol) was added and stirred in a nitrogen atmosphere. The flask was kept in the ice bath for 15 minutes until a rise in temperature of the reaction solution stopped. Thereafter, the reaction solution was stirred at 25°C for 4 hours. After stirring, 33% aqueous sodium hydroxide solution (5.7 g) was added to the reaction solution to stop the reaction. The organic layer taken out of the reaction solution was dried with anhydrous magnesium sulfate and concentrated, resulting in 6.2 g of colorless transparent liquid. This liquid was purified by column chromatography (using hexane : ethyl acetate = 99 : 1 as eluent). By charging 3.0 g of the liquid into the column chromatography, 2.7 g of 3,6-dimethylheptane-2-yl acetate (I-a-1) was obtained.

The following is the results of ¹H-NMR, and ¹³C-NMR measurements and the odor evaluation of 3,6-dimethylheptane-2-yl acetate (compound (I-a-1)).
¹H-NMR (CDCl₃, 400 MHz, δ ppm): 0.88 (d, 9H), 1.01-1.27 (m, 6H), 1.33-1.42 (m, 1H), 1.44-1.67 (m, 2H), 2.92 (s, 3H), 4.82 (m, 1H)
¹³C-NMR (CDCl₃, 400 MHz, δ ppm): 14.6 (CH₃), 14.8 (CH₃), 15.8 (CH₃), 16.9 (CH₃), 21.3 (CH₃), 21.4 (CH₃), 22.4 (CH₃), 22.8 (CH₃), 28.1 (CH₂), 28.2 (CH₂), 30.1 (CH), 30.3 (CH), 36.2 (CH₂), 36.3 (CH₂), 37.4 (CH), 37.7 (CH), 74.0 (CH), 74.2 (CH), 170.7 (C), 170.8 (C)

Odor evaluation: fruity, pear-like, green

### [Example 2]

### Production of 3,6-dimethylheptane-2-yl propionate (compound (I-a-2))

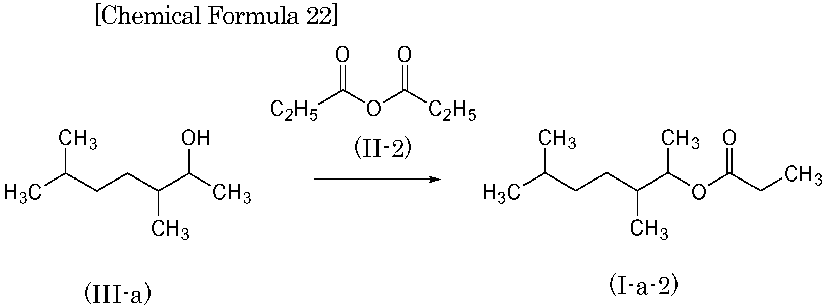

3,6-dimethylheptane-2-ol (compound (III-a)) (5.0 g, 34 mmol) produced in Production Example 2 and propionic anhydride (II-2) (5.4 g, 42 mmol) were placed in a flask and cooled in an ice bath. Then, DMAP (49 mg, 0.4 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 3 hours. After the reaction was finished, 33% aqueous sodium hydroxide solution (5.7 g) was added to the reaction solution to stop the reaction. The organic layer taken out of the reaction solution was dried with anhydrous magnesium sulfate and concentrated, resulting in 7.0 g of colorless transparent liquid. This liquid was purified by column chromatography (using hexane : ethyl acetate = 99 : 1 as eluent). By charging 3.0 g of the liquid into the column chromatography, 2.2 g of 3,6-dimethylheptane-2-yl propionate (I-a-2) was obtained.

The following is the results of ¹H-NNR and ¹³C-NMR measurements and the odor evaluation of 3,6-dimethylheptane-2-yl propionate (compound (I-a-2)).
¹H-NMR (CDCl₃, 400 MHz, δ ppm): 0.88 (d, 9H), 1.03-1.27 (m, 9H), 1.34-1.42 (m, 1H), 1.44-1.66 (m, 2H), 2.30 (q, 2H), 4.83 (m, 1H)
¹³C-NMR (CDCl₃, 400 MHz, δ ppm): 9.2 (CH₃), 9.3 (CH₃), 14.6 (CH₃), 14.8 (CH₃), 15.8 (CH₃), 16.9 (CH₃), 22.4 (CH₃), 22.7 (CH₃), 28.0 (CH₂), 28.1 (CH), 28.2 (CH), 30.1 (CH₂), 30.3 (CH₂), 36.2 (CH₂), 37.8 (CH₂), 37.5 (CH), 37.8 (CH), 73.7 (CH), 74.0 (CH), 174.0 (C), 174.1 (C)

Odor evaluation: fruity, pear-like, green

### [Example 3]

### Production of 3,6-dimethylheptane-2-yl n-butyrate (compound (I-a-3))

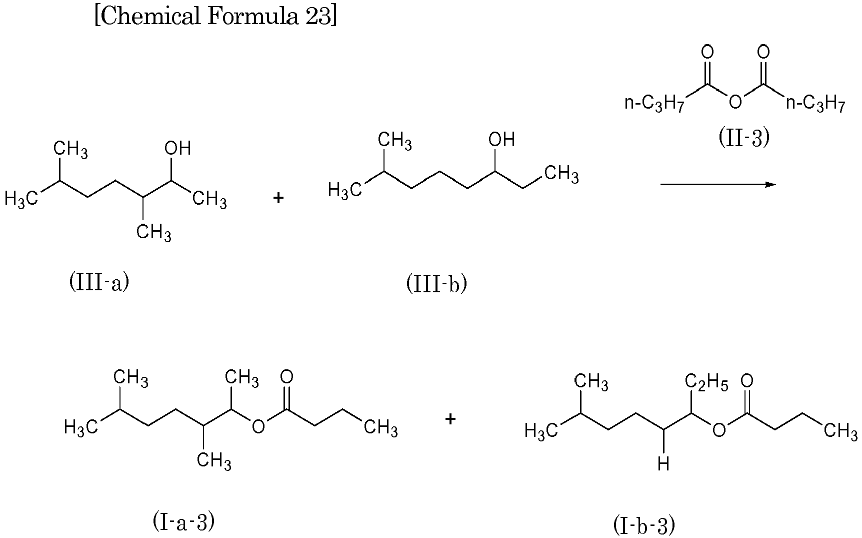

The mixture of 3,6-dimethylheptane-2-ol (compound (III-a)) and 7-methyloctane-3-ol (compound (III-b)) (compound (III-a) : compound (III-b) = 95 : 5, 5.0 g, 34 mmol) produced in Production Example 3 and n-butyric anhydride (II-3) (6.6 g, 42 mmol) were placed in a flask and cooled in an ice bath. Then, DMAP (49 mg, 0.4 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 3 hours. After the reaction was finished, 33% aqueous sodium hydroxide solution (5.7 g) was added to the reaction solution to stop the reaction. The organic layer taken out of the reaction solution was dried with anhydrous magnesium sulfate and concentrated, resulting in 7.5 g of colorless transparent liquid. This liquid was purified by column chromatography (using hexane : ethyl acetate = 99 : 1 as eluent). By charging 5.1 g of the liquid into the column chromatography, 2.3 g of 3,6-dimethylheptane-2-yl n-butyrate (I-a-3) was obtained.

The following is the results of ¹H-NMR, and ¹³C-NMR measurements and the odor evaluation of 3,6-dimethylheptane-2-yl n-butyrate (compound (I-a-3)).
¹H-NMR (CDCl₃, 400 MHz, δ ppm): 0.80 (dd, 8H), 0.95 (t, 3H), 1.01-1.22 (m, 6H), 1.35-1.40 (m, 1H), 1.48-1.53 (m, 2H), 1.60-1.68 (m, 3H), 2.26 (t, 2H), 4.84 (m, 1H)
¹³C-NMR (CDCl₃, 400 MHz, δ ppm): 13.7 (CH₃), 14.7 (CH₃), 14.8 (CH₃), 15.9 (CH₃), 17.0 (CH₃), 18.6 (CH₂), 18.7 (CH₂), 22.4 (CH₃), 22.8 (CH₃), 28.2 (CH), 28.2 (CH₂), 30.2 (CH₂), 30.3 (CH₂), 36.3 (CH₂), 36.4 (CH₂), 36.7 (CH₂), 37.5 (CH), 37.9 (CH), 73.7 (CH), 74.0 (CH), 173.3 (C), 173.4 (C)

Odor evaluation: fruity, floral

### [Example 4]

### Production of 3,6-dimethylheptane-2-yl isobutyrate (compound (I-a-4))

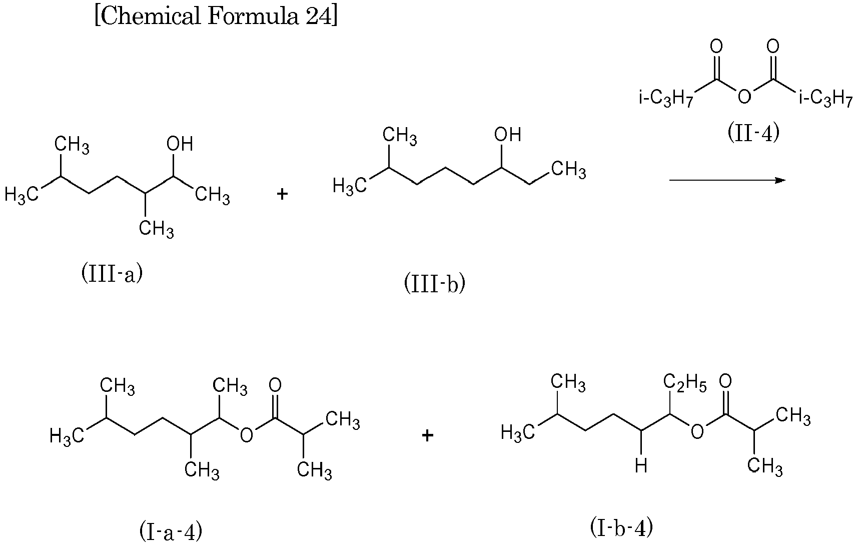

The mixture of 3,6-dimethylheptane-2-ol (compound (III-a)) and 7-methyloctane-3-ol (compound (III-b)) (compound (III-a) : compound (III-b) = 95 : 5, 5.0 g, 34 mmol) produced in Production Example 3 and isobutyric anhydride (II-4) (6.6 g, 42 mmol) were placed in a flask and cooled in an ice bath. Then, DMAP (49 mg, 0.4 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 3 hours. After the reaction was finished, 33% aqueous sodium hydroxide solution (5.7 g) was added to the reaction solution to stop the reaction. The organic layer taken out of the reaction solution was dried with anhydrous magnesium sulfate and concentrated, resulting in 7.4 g of colorless transparent liquid. This liquid was purified by column chromatography (using hexane : ethyl acetate = 99 : 1 as eluent). By charging 6.8 g of the liquid into the column chromatography, 2.1 g of 3,6-dimethylheptane-2-yl isobutyrate (I-a-4) was obtained.

The following is the results of ¹H-NMR, and ¹³C-NMR measurements and the odor evaluation of 3,6-dimethylheptane-2-yl isobutyrate (compound (I-a-4)).
¹H-NMR (CDCl₃, 400 MHz, δ ppm): 0.86-0.91 (m, 9H), 1.06-1.24 (m, 12H), 1.35-1.64 (m, 4H), 2.46-2.57 (m, 1H), 4.77-4.87 (m, 1H)
¹³C-NMR (CDCl₃, 400 MHz, δ ppm): 14.6 (CH₃), 14.8 (CH₃), 15.9 (CH₃), 17.0 (CH₃), 18.9 (CH₃), 19.1 (CH₂), 19.2 (CH₂), 22.4 (CH₃), 22.8 (CH₃), 28.1 (CH), 28.2 (CH), 30.3 (CH₂), 34.3 (CH), 34.4 (CH), 36.2 (CH₂), 36.4 (CH₂), 37.5 (CH), 37.9 (CH), 73.4 (CH), 74.8 (CH), 176.7 (C), 176.5 (C)

Odor evaluation: fruity, woody

### [Example 5] - Reference example

### Production of 7-methyloctane-3-yl acetate (compound (I-b-1))

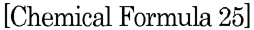

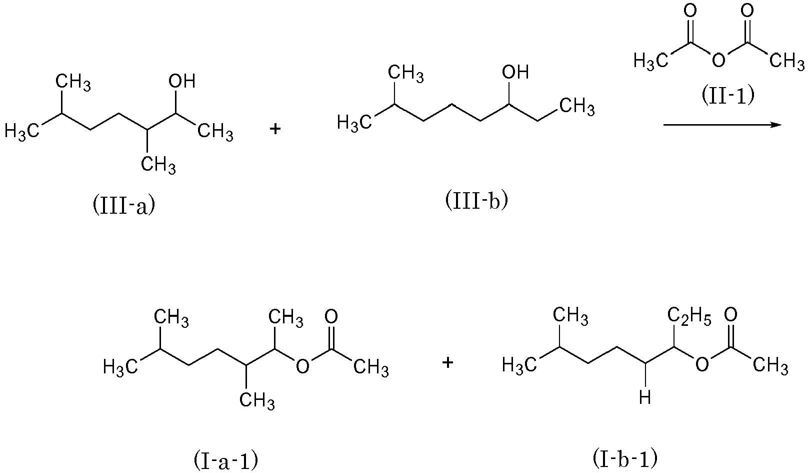

The mixture of 3,6-dimethylheptane-2-ol (compound (III-a)) and 7-methyloctane-3-ol (compound (III-b)) (compound (III-a) : compound (III-b) = 63 : 37, 5.0 g, 34 mmol) produced in Production Example 3 and acetic anhydride (II-1) (4 g, 41.7 mmol) were placed in a flask and cooled in an ice bath. Then, DMAP (51 mg, 0.4 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 5 hours. After the reaction was finished, 33% aqueous sodium hydroxide solution (5.0 g) was added to the reaction solution to stop the reaction. The organic layer taken out of the reaction solution was dried with anhydrous magnesium sulfate and concentrated, resulting in 6.3 g of colorless transparent liquid. This liquid was purified by column chromatography (hexane : ethyl acetate = 99 : 1). By charging 6 g of the liquid into the column chromatography, 0.56 g of a mixture of 7-methyloctane-3-yl acetate (I-b-1) and 3,6-dimethylheptane-2-yl acetate (I-a-1) (compound (I-b-1) : compound (I-a-1) = 85 : 15) was obtained. Moreover, 0.52 g of this mixture was purified by column chromatography (using hexane : ethyl acetate = 97 : 3 as eluent). Thus, 49 mg of 7-methyloctane-3-yl acetate (I-b-1) was obtained.

The following is the results of ¹H-NNR and ¹³C-NMR measurements and the odor evaluation of 7-methyloctane-3-yl acetate (compound (I-b-1)).
¹H-NMR (CDCl₃, 400 MHz, δ ppm): 0.86-1.11 (m, 9H), 1.13-1.21 (m, 2H), 1.23-1.33 (m, 2H), 1.47-1.60 (m, 2H), 2.05 (s, 3H), 4.80 (quin, 1H)
¹³C-NMR (CDCl₃, 400 MHz, δ ppm): 9.5 (CH₃), 21.2 (CH₃), 22.5 (CH₃), 22.5 (CH₃), 23.0 (CH₂), 26.9 (CH₂), 27.8 (CH), 33.8 (CH₂), 38.8 (CH₂), 75.5 (CH), 171.0 (C)

Odor evaluation: citrus, mandarin, fruity

### [Example 6] - Reference example

### Production of 7-methyloctane-3-yl propionate (compound (I-b-2))

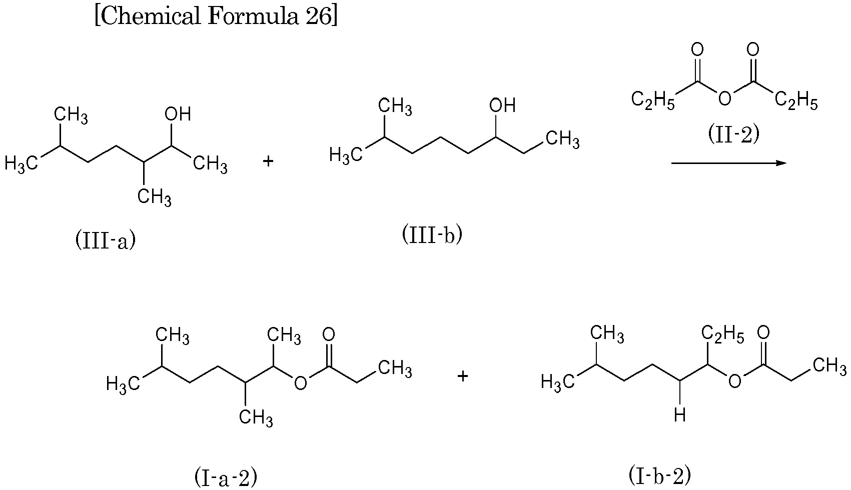

The mixture of 3,6-dimethylheptane-2-ol (compound (III-a)) and 7-methyloctane-3-ol (compound (III-b)) (compound (III-a) : compound (III-b) = 63 : 37, 5.0 g, 34 mmol) produced in Production Example 3 and propionic anhydride (II-2) (5.4 g, 41 mmol) were placed in a flask and cooled in an ice bath. Then, DMAP (51 mg, 0.44 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 5 hours. After the reaction was finished, 33% aqueous sodium hydroxide solution (5.0 g) was added to the reaction solution to stop the reaction. The organic layer taken out of the reaction solution was dried with anhydrous magnesium sulfate and concentrated, resulting in 6.3 g of colorless transparent liquid. This liquid was purified by column chromatography (hexane : ethyl acetate = 99 : 1). By charging 5.5 g of the liquid into the column chromatography, 0.72 g of a mixture of 7-methyloctane-3-yl propionate (I-b-2) and 3,6-dimethylheptane-2-yl propionate (I-a-2) (compound (I-b-2) : compound (I-a-2) = 89 : 11) was obtained. Moreover, 0.44 g of this mixture was purified by column chromatography (using hexane : ethyl acetate = 97 : 3 as eluent). Thus, 77 mg of 7-methyloctane-3-yl propionate (I-b-2) was obtained.

The following is the results of ¹H-NMR and ¹³C-NMR measurements and the odor evaluation of 7-methyloctane-3-yl propionate (compound (I-b-2)).
¹H-NMR (CDCl₃, 400 MHz, δ ppm): 0.85-0.90 (m, 9H), 1.11-1.19 (m, 5H), 1.21-1.33 (m, 2H), 1.47-1.62 (m, 5H), 2.31 (q, 2H), 4.82 (quin, 1H)
¹³C-NMR (CDCl₃, 400 MHz, δ ppm): 9.3 (CH₃), 9.6 (CH₃), 22.4 (CH₃), 22.5 (CH₃), 23.0 (CH₂), 27.0 (CH₂), 27.8 (CH), 27.9 (CH₂), 33.8 (CH₂), 38.8 (CH₂), 75.2 (CH), 174.3 (C)

Odor evaluation: fruity, strawberry

### [Example 7] - Reference example

### Production of 7-methyloctane-3-yl n-butyrate (compound (I-b-3))

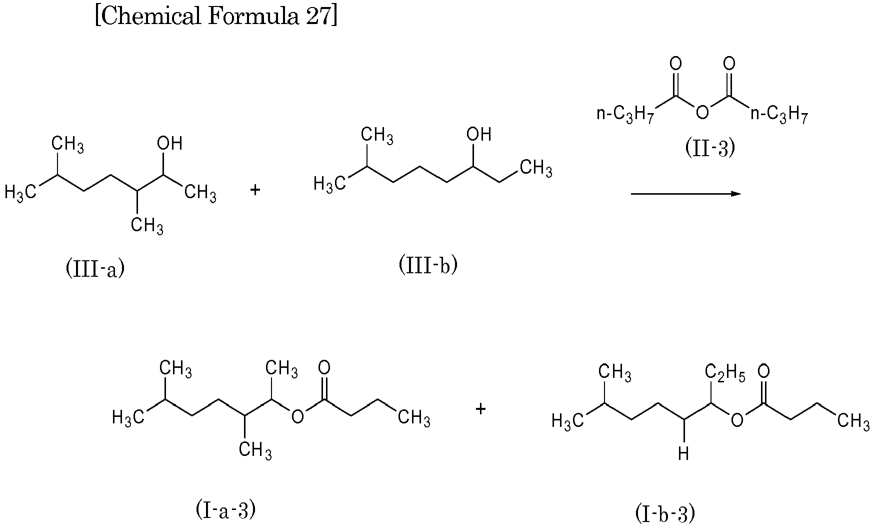

The mixture of 3,6-dimethylheptane-2-ol (compound (III-a)) and 7-methyloctane-3-ol (compound (III-b)) (compound (III-a) : compound (III-b) = 63 : 37, 5.0 g, 34 mmol) produced in Production Example 3 and n-butyric anhydride (II-3) (6.9 g, 42 mmol) were placed in a flask and cooled in an ice bath. Then, DMAP (51 mg, 0.44 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 5 hours. After the reaction was finished, 33% aqueous sodium hydroxide solution (5.6 g) was added to the reaction solution to stop the reaction. The organic layer taken out of the reaction solution was dried with anhydrous magnesium sulfate and concentrated, resulting in 6.9 g of colorless transparent liquid. This liquid was purified by column chromatography (hexane : ethyl acetate = 99 : 1). By charging 5.0 g of the liquid into the column chromatography, 1.2 g of a mixture of 7-methyloctane-3-yl n-butyrate (I-b-3) and 3,6-dimethylheptane-2-yl n-butyrate (I-a-3) (compound (I-b-3) : compound (I-a-3) = 94 : 6) was obtained. Moreover, 0.46 g of this mixture was purified by column chromatography (using hexane : ethyl acetate = 97 : 3 as eluent). Thus, 79 mg of 7-methyloctane-3-yl n-butyrate (I-b-3) was obtained.

The following is the results of ¹H-NMR, and ¹³C-NMR measurements and the odor evaluation of 7-methyloctane-3-yl n-butyrate (compound (I-b-3)).
¹H-NMR (CDCl₃, 400 MHz, δ ppm): 0.85-0.90 (d, 9H), 0.96 (t, 3H), 1.13-1.21 (m, 2H), 1.23-1.33 (m, 2H), 1.47-1.60 (m, 5H), 1.62-1.71 (m, 2H), 2.28 (t, 2H), 4.83 (quin, 1H)
¹³C-NMR (CDCl₃, 400 MHz, δ ppm): 9.6 (CH₃), 13.7 (CH₃), 18.7 (CH₂), 22.5 (CH₃), 22.6 (CH₃), 23.1 (CH₂), 27.0 (CH₂), 27.9 (CH), 33.9 (CH₂), 36.6 (CH₂), 38.8 (CH₂), 75.2 (CH), 173.6 (C)

Odor evaluation: fruity

### [Example 8] - Reference example

### Production of 7-methyloctane-3-yl isobutyrate (compound (I-b-4))

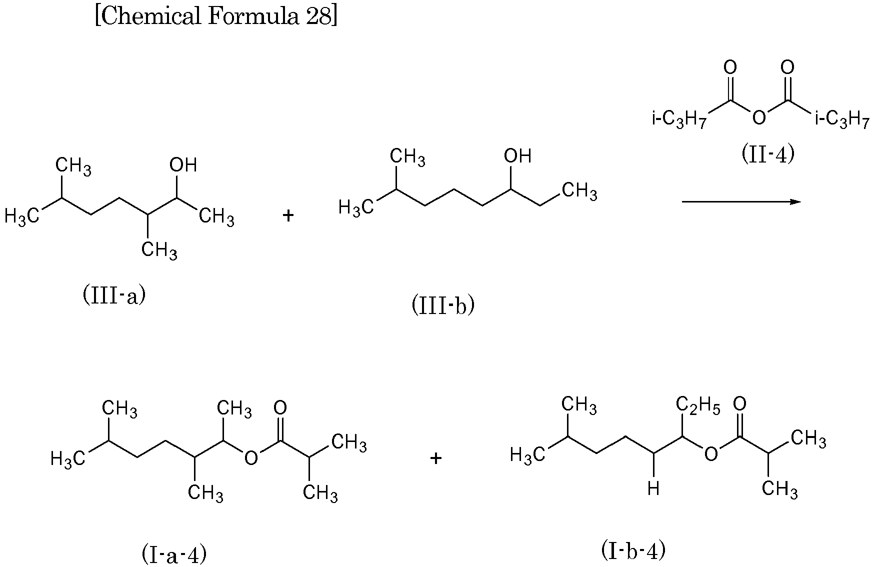

The mixture of 3,6-dimethylheptane-2-ol (compound (III-a)) and 7-methyloctane-3-ol (compound (III-b)) (compound (III-a) : compound (III-b) = 63 : 37, 5.0 g, 34 mmol) produced in Production Example 3 and isobutyric anhydride (II-4) (6.7 g, 42 mmol) were placed in a flask and cooled in an ice bath. Then, DMAP (51 mg, 0.44 mmol) was added and stirred in a nitrogen atmosphere. The flask was kept in the ice bath for 10 minutes until a rise in temperature of the reaction solution stopped. Thereafter, the reaction solution was stirred at a normal temperature of 25°C for 5 hours. After the reaction was finished, 33% aqueous sodium hydroxide solution (5.0 g) was added to the reaction solution to stop the reaction. The organic layer taken out of the reaction solution was dried with anhydrous magnesium sulfate and concentrated, resulting in 6.0 g of colorless transparent liquid. By charging 5.0 g of the liquid into the column chromatography, 1.1 g of a mixture of 7-methyloctane-3-yl isobutyrate (I-b-4) and 3,6-dimethylheptane-2-yl isobutyrate (I-a-4) (compound (I-b-4) : compound (I-a-4) = 88 : 12) was obtained. Moreover, 0.46 g of this mixture was purified by column chromatography (using hexane : ethyl acetate = 97 : 3 as eluent). Thus, 29 mg of 7-methyloctane-3-yl isobutyrate (I-b-4) was obtained.

The following is the results of ¹H-NMR and ¹³C-NMR measurements and the odor evaluation of 7-methyloctane-3-yl isobutyrate (compound (I-b-4)).
¹H-NMR (CDCl₃, 400 MHz, δ ppm): 0.85-0.91 (d, 9H), 1.09-1.22 (m, 8H), 1.23-1.33 (m, 2H), 1.47-1.61 (m, 5H), 2.53 (sep, 1H), 4.81 (quin, 1H)
¹³C-NMR (CDCl₃, 400 MHz, δ ppm): 9.53 (CH₃), 19.1 (2CH₃), 22.4 (CH₃), 22.5 (CH₃), 23.0 (CH₂), 26.7 (CH₂), 27.8 (CH), 33.8 (CH₂), 34.3 (CH), 38.8 (CH₂), 74.9 (CH), 177.0 (C)

Odor evaluation: fruity, herbal

**[TABLE 1]**

| | | | | | | | | | | | |
|---|---|---|---|---|---|---|---|---|---|---|---|
| Comparative Example 1 → 1-octene-3-yl acetate | | | | | | | | | | | |
| Comparative Example 2 → tetrahydrolinalyl acetate | | | | | | | | | | | |
| Comparative Example 3 → nonyl acetate | | | | | | | | | | | |

| | Example | | | | Reference Example | | | | Comparative Example | | |
|---|---|---|---|---|---|---|---|---|---|---|---|
| | 1 | 2 | 3 | 4 | 5 | 6 | 7 | 8 | 1 | 2 | 3 |
| Compound number | I-a-1 | I-a-2 | I-a-3 | I-a-4 | I-b-1 | I-b-2 | I-b-3 | I-b-4 | - | - | - |
| Freshness | present | present | present | present | present | present | present | present | absent | present | absent |
| Assessment | 4 | 4 | 3 | 3 | 4 | 3 | 3 | 2 | 1 | 3 | 1 |
| Odor | fruity | fruity | fruity | fruity | citrus | fruity | fruity | fruity | herbal | floral | fruity |
| | pear-like | pear-like | floral | woody | mandarin | strawberry | - | herbal | mint | fruity | oil-like |
| | green | green | - | - | fruity | - | - | - | fruity | | |

### [Example 9]

0.88 mg of 6-dimethylheptane-2-yl acetate (I-a-1) produced in Example 1 was diluted with acetone up to 10 % by mass. 14 mg of the mixture of 7-methyloctane-3-yl acetate (I-b-1) and 3,6-dimethylheptane-2-yl acetate (I-a-1) (compound (I-b-1) : compound (I-a-1) = 85 : 15) produced in Example 5 was diluted with acetone up to 10 % by mass. The diluent of the compound (I-a-1) was referred to as a diluent A. The diluent of the mixture (compound (I-b-1) : compound (I-a-1) = 85 : 15) was referred to as a diluent B. Then, 8.8 mg of the diluent A and 140 mg of the diluent B were mixed to form a mixture of 3,6-dimethylheptane-2-yl acetate (I-a-1) and 7-methyloctane-3-yl acetate (I-b-1) (compound (I-a-1) : compound (I-b-1) = 95 : 5).

### [Example 10]

2.6 mg of 6-dimethylheptane-2-yl acetate (I-a-1) produced in Example 1 was diluted with acetone up to 10 % by mass. 12 mg of the mixture of 7-methyloctane-3-yl acetate (I-b-1) and 3,6-dimethylheptane-2-yl acetate (I-a-1) (compound (I-b-1) : compound (I-a-1) = 85 : 15) produced in Example 5 was diluted with acetone up to 10 % by mass. The diluent of the compound (I-a-1) was referred to as a diluent C. The diluent of the mixture (compound (I-b-1) : compound (I-a-1) = 85 : 15) was referred to as a diluent D. Then, 26 mg of the diluent C and 120 mg of the diluent D were mixed to form a mixture of 3,6-dimethylheptane-2-yl acetate (I-a-1) and 7-methyloctane-3-yl acetate (I-b-1) (compound (I-a-1) : compound (I-b-1) = 85 : 15).

### [Example 11]

4.4 mg of 6-dimethylheptane-2-yl acetate (I-a-1) produced in Example 1 was diluted with acetone up to 10 % by mass. 11 mg of the mixture of 7-methyloctane-3-yl acetate (I-b-1) and 3,6-dimethylheptane-2-yl acetate (I-a-1) (compound (I-b-1) : compound (I-a-1) = 85 : 15) produced in Example 5 was diluted with acetone up to 10 % by mass. The diluent of the compound (I-a-1) was referred to as a diluent E. The diluent of the mixture (compound (I-b-1) : compound (I-a-1) = 85 : 15) was referred to as a diluent F. Then, 44 mg of the diluent E and 110 mg of the diluent F were mixed to form a mixture of 3,6-dimethylheptane-2-yl acetate (I-a-1) and 7-methyloctane-3-yl acetate (I-b-1) (compound (I-a-1) : compound (I-b-1) = 75 : 25).

Table 2 shows the sensory evaluation of the mixtures produced in Examples 25 to 27.

**[TABLE 2]**

| | | Example 9 | Example 10 | Example 11 |
|---|---|---|---|---|
| Mixing ratio | Compound (I-a-1) | 95 | 85 | 75 |
| | Compound (I-b-1) | 5 | 15 | 25 |
| Freshness | | present | present | present |
| Assessment | | 4 | 4 | 3 |
| Odor | | fruity | fruity | fruity |
| | | pear-like | pear-like | mandarin |
| | | green | green or mandarin | |

As shown in Tables 1 and 2, the results confirmed that the esters of the present invention had a main odor with a fresh and fruity note.

### [Examples 12 to 19 (softener)]

The esters produced in Examples 1 to 8 were used to prepare softeners, each of which had a composition shown in Table 2, and the odor evaluation was performed. The results are expected to show that the softeners can provide a fresh and fruity feeling, as shown in Table 3.

**[TABLE 3]**

| | | Example | | | | | | | |
|---|---|---|---|---|---|---|---|---|---|
| | | 12 | 13 | 14 | 15 | 16 | 17 | 18 | 19 |
| Softener | N,N-di[2-(alkanoyloxy)ethyl]-N-(2-hydroxyethyl) -N-methyl ammonium sulfate | 15 | 15 | 15 | 15 | 15 | 15 | 15 | 15 |
| | Stearic acid | 0.5 | 0.5 | 0.5 | 0.5 | 0.5 | 0.5 | 0.5 | 0.5 |
| | Calcium chloride | 0.05 | 0.05 | 0.05 | 0.05 | 0.05 | 0.05 | 0.05 | 0.05 |
| | Ethanol | 2 | 2 | 2 | 2 | 2 | 2 | 2 | 2 |
| | Ester | 0.2 | 0.2 | 0.2 | 0.2 | 0.2 | 0.2 | 0.2 | 0.2 |
| | Ion exchanged water | suitable amount | suitable amount | suitable amount | suitable amount | suitable amount | suitable amount | suitable amount | suitable amount |
| | pH adjuster | suitable amount | suitable amount | suitable amount | suitable amount | suitable amount | suitable amount | suitable amount | suitable amount |
| | Total (% by mass) | 100 | 100 | 100 | 100 | 100 | 100 | 100 | 100 |
| | pH | 4 | 4 | 4 | 4 | 4 | 4 | 4 | 4 |
| Ester | Compound number | I-a-1 | I-a-2 | I-a-3 | I-a-4 | I-b-1 | I-b-2 | I-b-3 | I-b-4 |
| Evaluation | Odor | fruity | fruity | fruity | fruity | citrus | fruity | fruity | fruity |
| | | pear-like | pear-like | floral | woody | mandarin | strawberry | - | herbal |
| | | green | green | - | - | fruity | - | - | - |
| | Assessment | excellent fresh feeling | excellent fresh feeling | good fresh feeling | good fresh feeling | excellent fresh feeling | good fresh feeling | good fresh feeling | fresh feeling |

### [Examples 20 to 23 and Comparative Examples 4 to 8 (fragrance composition for liquid detergent for clothing)]

The fragrance compositions containing the esters produced in Examples 1 to 8 were used to prepare fragrance compositions for a white floral-type powder detergent for clothing. Each of the fragrance compositions had a composition shown in Table 4. For comparative examples, tetrahydrolinalyl acetate, 1-octene-3-yl acetate, nonyl acetate, and isononyl acetate were used to prepare fragrance compositions for a white floral-type liquid detergent for clothing. Each of the fragrance compositions had a composition shown in Table 4. The odor evaluation was performed in the same manner as Example 1, and odors were determined when the fragrance compositions were used for a liquid detergent for clothing. Table 5 shows the results of the evaluation.

**[TABLE 4]**

| | Comp. Ex. 4 | Example | | | | Comparative Example | | | |
|---|---|---|---|---|---|---|---|---|---|
| | | 20 | 21 | 22 | 23 | 5 | 6 | 7 | 8 |
| Dihydromyrcenol | 50 | 50 | 50 | 50 | 50 | 50 | 50 | 50 | 50 |
| Triplal¹⁾ | 4 | 4 | 4 | 4 | 4 | 4 | 4 | 4 | 4 |
| Magnol²⁾ | 2 | 2 | 2 | 2 | 2 | 2 | 2 | 2 | 2 |
| Undecavertol³⁾ | 2 | 2 | 2 | 2 | 2 | 2 | 2 | 2 | 2 |
| o-Tert-butyl cyclohexyl acetate | 20 | 20 | 20 | 20 | 20 | 20 | 20 | 20 | 20 |
| Poirenate⁴⁾ | 2 | 2 | 2 | 2 | 2 | 2 | 2 | 2 | 2 |
| Raspberry ketone | 5 | 5 | 5 | 5 | 5 | 5 | 5 | 5 | 5 |
| Fruitate⁵⁾ | 2 | 2 | 2 | 2 | 2 | 2 | 2 | 2 | 2 |
| Tricyclodecenyl propionate | 100 | 100 | 100 | 100 | 100 | 100 | 100 | 100 | 100 |
| Geraniol | 100 | 100 | 100 | 100 | 100 | 100 | 100 | 100 | 100 |
| Rose oxide | 1 | 1 | 1 | 1 | 1 | 1 | 1 | 1 | 1 |
| α-Hexyl cinnamaldehyde | 100 | 100 | 100 | 100 | 100 | 100 | 100 | 100 | 100 |
| Methyl dihydrojasmonate | 50 | 50 | 50 | 50 | 50 | 50 | 50 | 50 | 50 |
| Dimethyl anthranilate | 5 | 5 | 5 | 5 | 5 | 5 | 5 | 5 | 5 |
| Methyl-β-naphthyl ketone | 5 | 5 | 5 | 5 | 5 | 5 | 5 | 5 | 5 |
| Lilial⁶⁾ | 100 | 100 | 100 | 100 | 100 | 100 | 100 | 100 | 100 |
| Linalool | 50 | 50 | 50 | 50 | 50 | 50 | 50 | 50 | 50 |
| Terpineol | 50 | 50 | 50 | 50 | 50 | 50 | 50 | 50 | 50 |
| Phenylacetaldehyde dimethyl acetal | 4 | 4 | 4 | 4 | 4 | 4 | 4 | 4 | 4 |
| Amber Core⁷⁾ | 30 | 30 | 30 | 30 | 30 | 30 | 30 | 30 | 30 |
| Isobornylcyclohexanol | 20 | 20 | 20 | 20 | 20 | 20 | 20 | 20 | 20 |
| Galaxolide 50%IPM⁸⁾ | 60 | 60 | 60 | 60 | 60 | 60 | 60 | 60 | 60 |
| Ambroxan⁹⁾ 5%DPG¹⁰⁾ | 4 | 4 | 4 | 4 | 4 | 4 | 4 | 4 | 4 |
| Heliotropyl acetone | 1 | 1 | 1 | 1 | 1 | 1 | 1 | 1 | 1 |
| I-a-1 | - | 47.5 | - | - | - | - | - | - | - |
| I-b-1 | - | 2.5 | - | - | - | - | - | - | - |
| I-a-2 | - | - | 47.5 | - | - | - | - | - | - |
| I-b-2 | - | - | 2.5 | - | - | - | - | - | - |
| I-a-3 | - | - | - | 47.5 | - | - | - | - | - |
| I-b-3 | - | - | - | 2.5 | - | - | - | - | - |
| I-a-4 | - | - | - | - | 47.5 | - | - | - | - |
| I-b-4 | - | - | - | - | 2.5 | - | - | - | - |
| Tetrahydrolinalyl acetate | - | - | - | - | - | 50 | - | - | - |
| 1-Octene-3-yl acetate | - | - | - | - | - | - | 50 | - | - |
| Nonyl acetate | - | - | - | - | - | - | - | 50 | - |
| Isononyl acetate | - | - | - | - | - | - | - | - | 50 |
| Dipropylene glycol | 233 | 183 | 183 | 183 | 183 | 183 | 183 | 183 | 183 |
| Total | 1000 | 1000 | 1000 | 1000 | 1000 | 1000 | 1000 | 1000 | 1000 |

| | | | | | | | | | |
|---|---|---|---|---|---|---|---|---|---|
| [0217] 1) TRIPLAL: IFF (Trade Name), compound name: 2,4-dimethyl-3-cyclohexene-1-carboxaldehyde 2) MAGNOL: Kao Corporation (Trade Name), a mixture containing ethyl norbornyl cyclohexanol as a main component 3) UNDECAVERTOL: Givaudan (Trade Name), compound name: 4-methyl-3-decene-5-ol 4) POIRENATE: Kao Corporation (Trade Name), compound name: ethyl 2-cyclohexyl propionate 5) FRUITATE: Kao Corporation (Trade Name), compound name: ethyl tricyclo[5.2.1.0^{2.6}]decan-2-carboxylate 6) LILIAL: Givaudan (Trade Name), compound name: p-tert-butyl-α-methyl hydrocinnamaldehyde 7) AMBER CORE: Kao Corporation (Trade Name), compound name: 1-(2-tert-butylcyclohexyloxy)-2-butanol 8) 50%IPM: 50% isopropyl myristate (IPM) solution 9) AMBROXAN: Kao Corporation (Trade Name), compound name: dodecahydro-3a,6,6,9a-tetramethyl naphtho[2.1-b]furan 10) 5%DPG: 5% dipropylene glycol (DPG) solution | | | | | | | | | |

**[TABLE 5]**

| Comparative Example 4 | Bright white floral odor |
|---|---|
| Example 20 | A fresh and dewy feeling was added to increase the intensity of the odor. |
| Example 21 | A fresh and dewy feeling was added to increase the intensity of the odor. |
| Example 22 | A fresh and dewy feeling was added to increase the intensity of the odor. Compared to Examples 20 and 21, the odor gave a somewhat heavy impression. |
| Example 23 | A fresh and dewy feeling was added to increase the intensity of the odor. Compared to Examples 20 and 21, the odor gave a somewhat heavy impression. |
| Comparative Example 5 | A sweet and fruity feeling was added. |
| Comparative Example 6 | Heavy earthy odor with poor brightness |
| Comparative Example 7 | The odor gave a heavy impression and reduced brightness. |
| Comparative Example 8 | A fruity feeling was added. |

As shown in Table 5, the results confirmed that the fragrance compositions containing the esters of the present invention had a fresh and fruity note.

### [Examples 24 to 27 and Comparative Examples 9 to 13 (fragrance composition for body cream)]

The fragrance compositions containing the esters produced in Examples 1 to 8 were used to prepare fragrance compositions for a fruity pear-type body cream. Each of the fragrance compositions had a composition shown in Table 6. For comparative examples, tetrahydrolinalyl acetate, 1-octene-3-yl acetate, nonyl acetate, and isononyl acetate were used to prepare fragrance compositions for a fruity pear-type body cream. Each of the fragrance compositions had a composition shown in Table 6. The odor evaluation was performed in the same manner as Example 1, and odors were determined by the sensory evaluation when the fragrance compositions were used for a body cream. Table 6 shows the results of the sensory evaluation.

**[TABLE 6]**

| | Comp. Ex. 9 | Example | | | | Comparative Example | | | |
|---|---|---|---|---|---|---|---|---|---|
| | | 24 | 25 | 26 | 27 | 10 | 11 | 12 | 13 |
| Peranat¹⁾ | 25 | 25 | 25 | 25 | 25 | 25 | 25 | 25 | 25 |
| Cis-3-hexenol | 50 | 50 | 50 | 50 | 50 | 50 | 50 | 50 | 50 |
| Hexyl acetate | 100 | 100 | 100 | 100 | 100 | 100 | 100 | 100 | 100 |
| Liffarome²⁾ | 50 | 50 | 50 | 50 | 50 | 50 | 50 | 50 | 50 |
| Pollenal II³⁾ | 100 | 100 | 100 | 100 | 100 | 100 | 100 | 100 | 100 |
| Ethyl 2-methyl butyrate | 100 | 100 | 100 | 100 | 100 | 100 | 100 | 100 | 100 |
| Poirenate⁴⁾ | 150 | 150 | 150 | 150 | 150 | 150 | 150 | 150 | 150 |
| γ-Undecalactone | 150 | 150 | 150 | 150 | 150 | 150 | 150 | 150 | 150 |
| Melusat⁵⁾ | 100 | 100 | 100 | 100 | 100 | 100 | 100 | 100 | 100 |
| Nerolidol | 75 | 75 | 75 | 75 | 75 | 75 | 75 | 75 | 75 |
| Jasmacyclat⁶⁾ | 10 | 10 | 10 | 10 | 10 | 10 | 10 | 10 | 10 |
| I-a-1 | - | 47.5 | - | - | - | - | - | - | - |
| I-b-1 | - | 2.5 | - | - | - | - | - | - | - |
| I-a-2 | - | - | 47.5 | - | - | - | - | - | - |
| I-b-2 | - | - | 2.5 | - | - | - | - | - | - |
| I-a-3 | - | - | - | 47.5 | - | - | - | - | - |
| I-b-3 | - | - | - | 2.5 | - | - | - | - | - |
| I-a-4 | - | - | - | - | 47.5 | - | - | - | - |
| I-b-4 | - | - | - | - | 2.5 | - | - | - | - |
| Tetrahydrolinalyl acetate | - | - | - | - | - | 50 | - | - | - |
| 1-Octene-3-yl acetate | - | - | - | - | - | - | 50 | - | - |
| Nonyl acetate | - | - | - | - | - | - | - | 50 | - |
| Isononyl acetate | - | - | - | - | - | - | - | - | 50 |
| Dipropylene glycol | 90 | 40 | 40 | 40 | 40 | 40 | 40 | 40 | 40 |
| Total | 1000 | 1000 | 1000 | 1000 | 1000 | 1000 | 1000 | 1000 | 1000 |

| | | | | | | | | | |
|---|---|---|---|---|---|---|---|---|---|
| [0222] 1) PERANAT: Kao Corporation (Trade Name), compound name: 2-methylpentyl 2-methylvalerate 2) LIFFAROME: IFF (Trade Name), compound name: cis-3-hexenyl methyl carbonate 3) POLLENAL II: Kao Corporation (Trade Name), compound name: 2-cyclohexyl propanal 4) POIRENATE: Kao Corporation (Trade Name), compound name: ethyl 2-cyclohexyl propionate 5) MELUSAT: Kao Corporation (Trade Name), compound name: ethyl 3,5,5-trimethyl hexanoate 6) JASMACYCLAT: Kao Corporation (Trade Name), compound name: methyl cyclooctyl carbonate | | | | | | | | | |

**[TABLE 7]**

| Comparative Example 9 | Fruity pear-like odor with green feeling |
|---|---|
| Example 24 | A fresh feeling was added and a green feeling was more prominent. |
| Example 25 | A fresh feeling was added and a green feeling was more prominent. |
| Example 26 | A fresh feeling was added and a green feeling was more prominent. Compared to Examples 24 and 25, the odor gave a somewhat heavy and fruity impression. |
| Example 27 | A fresh feeling was added and a green feeling was more prominent. Compared to Examples 24 and 25, the odor gave a somewhat heavy and fruity impression. |
| Comparative Example 10 | The odor gave a sweet, soft, and fruity impression. |
| Comparative Example 11 | The odor was heavy and not very obvious. |
| Comparative Example 12 | The odor was heavy and not very obvious. |
| Comparative Example 13 | The odor was heavy and not very obvious. |

As shown in Table 7, the results confirmed that the fragrance compositions containing the esters of the present invention had a fresh and fruity note.

### Industrial Applicability

The ester of the present invention can be used as a fragrance material having a fresh and fruity fragrance note. Moreover, the ester of the present invention can be used alone or in combination with other components as a fragrance-imparting component for toiletry products such as hair cosmetics and softeners.

## Claims

1. An ester represented by the formula (I): 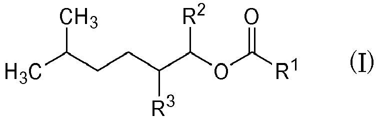 (in the formula, R¹ represents methyl, ethyl, propyl, or isopropyl, R² represents methyl, and R³ represents methyl)

2. The ester according to claim 1, wherein R¹ is methyl or ethyl.

3. A fragrance composition comprising the ester according to any one of claims 1 or 2.

4. The fragrance composition according to claim 3, wherein a content of the ester according to any one of claims 1 or 2 in the fragrance composition is 0.01 to 100 % by mass.

5. A fragrance composition comprising the ester according to claim 1 and the ester represented by formula (I-b):

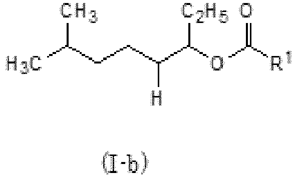

6. The fragrance composition according to claim 5, wherein a mass ratio of the ester according to claim 1 to the ester represented by formula (I-b) (the ester according to claim 1/ the ester represented by formula (I-b)) is 70/30 to 99.9/0.1.

7. The fragrance composition according to any one of claims 3 to 6, further comprising at least one selected from the group consisting of esters other than the ester represented by the formula (I), alcohols, carbonates, aldehydes, ketones, ethers, and lactones.

8. The fragrance composition according to any one of claims 3 to 7 further comprising an oil.

9. A fabric treatment composition comprising the fragrance composition according to any one of claims 3 to 8.

10. A cleaner composition comprising the fragrance composition according to any one of claims 3 to 8.

11. A cosmetic composition comprising the fragrance composition according to any one of claims 3 to 8.

12. A use of the ester according to any one of claims 1 or 2 as a fragrance-imparting component.

13. A method for producing an ester represented by the formula (I) of claim 1, comprising esterification of an alcohol represented by the formula (III) with a carboxylic anhydride represented by the formula (II): 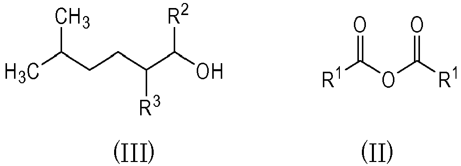 (in the formula, R¹ represents methyl, ethyl, propyl, or isopropyl, R² represents methyl and R³ represents methyl)

## Patentansprüche

1. Ester der Formel (I): 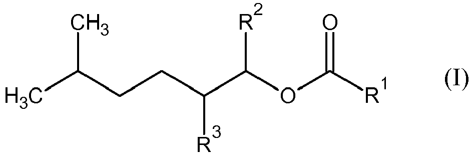 (worin, in der Formel, R¹ Methyl, Ethyl, Propyl oder Isopropyl bedeutet, R² Methyl bedeutet und R³ Methyl bedeutet)

2. Ester gemäß Anspruch 1, wobei R¹ Methyl oder Ethyl ist.

3. Duftstoffzusammensetzung, umfassend den Ester gemäß mindestens einem der Ansprüche 1 oder 2.

4. Duftstoffzusammensetzung gemäß Anspruch 3, wobei ein Gehalt des Esters gemäß mindestens einem der Ansprüche 1 oder 2 in der Duftstoffzusammensetzung 0,01 bis 100 Massen-% beträgt.

5. Duftstoffzusammensetzung, umfassend den Ester gemäß Anspruch 1 und einen Ester der Formel (I-b):

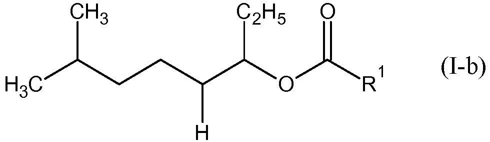

6. Duftstoffzusammensetzung gemäß Anspruch 5, wobei ein Massenverhältnis des Esters gemäß Anspruch 1 zu dem Ester der Formel (I-b) (der Ester gemäß Anspruch 1/der Ester der Formel (I-b)) 70/30 bis 99,9/0,1 beträgt.

7. Duftstoffzusammensetzung gemäß mindestens einem der Ansprüche 3 bis 6, ferner umfassend mindestens einen Vertreter, ausgewählt aus der Gruppe bestehend aus anderen Estern als dem Ester der Formel (I), Alkoholen, Carbonaten, Aldehyden, Ketonen, Ethern und Lactonen.

8. Duftstoffzusammensetzung gemäß mindestens einem der Ansprüche 3 bis 7, ferner umfassend ein Öl.

9. Textilbehandlungszusammensetzung, umfassend die Duftstoffzusammensetzung gemäß mindestens einem der Ansprüche 3 bis 8.

10. Reinigungsmittelzusammensetzung, umfassend die Duftstoffzusammensetzung gemäß mindestens einem der Ansprüche 3 bis 8.

11. Kosmetische Zusammensetzung, umfassend die Duftstoffzusammensetzung gemäß mindestens einem der Ansprüche 3 bis 8.

12. Verwendung des Esters gemäß mindestens einem der Ansprüche 1 oder 2 als eine Duft-verleihende Komponente.

13. Verfahren zur Herstellung eines Esters der Formel (I) nach Anspruch 1, umfassend die Veresterung eines Alkohols der Formel (III) mit einem Carbonsäureanhydrid der Formel (II): 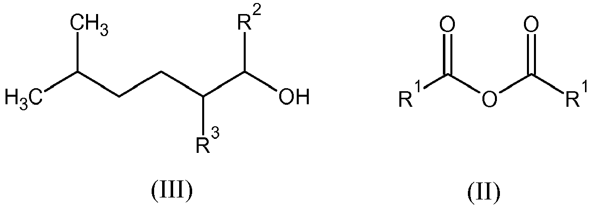 (worin, in der Formel, R¹ Methyl, Ethyl, Propyl oder Isopropyl bedeutet, R² Methyl bedeutet und R³ Methyl bedeutet).

## Revendications

1. Ester représenté par la formule (I) : 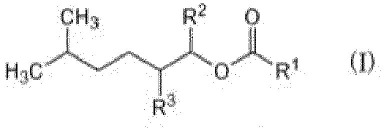 (dans la formule, R¹ représente un méthyle, un éthyle, un propyle, ou un isopropyle, R² représente un méthyle, et R³ représente un méthyle)

2. Ester selon la revendication 1, dans lequel R¹ est un méthyle ou un éthyle.

3. Composition de parfum comprenant l'ester selon l'une quelconque des revendications 1 ou 2.

4. Composition de parfum selon la revendication 3, dans laquelle une teneur en ester selon l'une quelconque des revendications 1 ou 2 dans la composition de parfum est de 0,01 à 100 % en masse.

5. Composition de parfum comprenant l'ester selon la revendication 1 et l'ester représenté par la formule (I-b) :

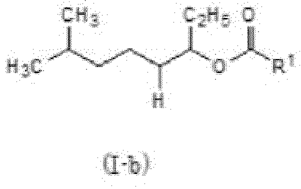

6. Composition de parfum selon la revendication 5, dans laquelle un rapport massique de l'ester selon la revendication 1 à l'ester représenté par la formule (I-b) (l'ester selon la revendication 1/l'ester représenté par la formule (I-b)) est de 70/30 à 99,9/0,1.

7. Composition de parfum selon l'une quelconque des revendications 3 à 6, comprenant en outre au moins un choisi dans le groupe constitué par les esters autres que l'ester représenté par la formule (I), les alcools, les carbonates, les aldéhydes, les cétones, les éthers, et les lactones.

8. Composition de parfum selon l'une quelconque des revendications 3 à 7 comprenant en outre une huile.

9. Composition de traitement du tissu comprenant la composition de parfum selon l'une quelconque des revendications 3 à 8.

10. Composition nettoyante comprenant la composition de parfum selon l'une quelconque des revendications 3 à 8.

11. Composition cosmétique comprenant la composition de parfum selon l'une quelconque des revendications 3 à 8.

12. Utilisation de l'ester selon l'une quelconque des revendications 1 ou 2 en tant que composant conférant un parfum.

13. Procédé pour la production d'un ester représenté par la formule (I) selon la revendication 1, comprenant l'estérification d'un alcool représenté par la formule (III) avec un anhydride carboxylique représenté par la formule (II) : 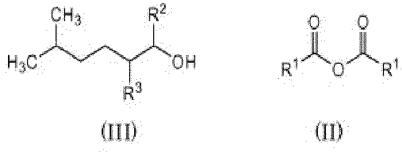 (dans la formule, R¹ représente un méthyle, un éthyle, un propyle, ou un isopropyle, R² représente un méthyle et R³ représente un méthyle)
